(19) Europäisches Patentamt / European Patent Office / Office européen des brevets

(11) **EP 2 156 846 B1**

(12) **EUROPEAN PATENT SPECIFICATION**


(45) Date of publication and mention of the grant of the patent: **13.08.2014 Bulletin 2014/33**

(21) Application number: **08752258.7**

(22) Date of filing: **01.05.2008**

(51) Int Cl.:
*A61K 45/00* (2006.01)
*A61K 31/137* (2006.01)
*A61K 38/00* (2006.01)
*A61P 1/12* (2006.01)
*C07K 5/027* (2006.01)

(86) International application number: **PCT/JP2008/058328**

(87) International publication number: **WO 2008/139947 (20.11.2008 Gazette 2008/47)**


(54) **PROPHYLACTIC OR THERAPEUTIC AGENT FOR DIARRHEA**

PROPHYLAKTISCHES ODER THERAPEUTISCHES MITTEL GEGEN DIARRHÖ

AGENT PROPHYLACTIQUE OU THÉRAPEUTIQUE CONTRE LA DIARRHÉE


(84) Designated Contracting States: **AT BE BG CH CY CZ DE DK EE ES FI FR GB GR HR HU IE IS IT LI LT LU LV MC MT NL NO PL PT RO SE SI SK TR**

(30) Priority: **08.05.2007 JP 2007123765**

(43) Date of publication of application: **24.02.2010 Bulletin 2010/08**



(73) Proprietor: **Ajinomoto Co., Inc.**
**Tokyo 104-8315 (JP)**

(72) Inventors:
- **YONEDA, Junya**
**Kawasaki-shi**
**Kanagawa 210-8681 (JP)**
- **YANO, Tetsuo**
**Kawasaki-shi**
**Kanagawa 210-8681 (JP)**
- **SEKI, Yukie**
**Kawasaki-shi**
**Kanagawa 210-8681 (JP)**
- **ETO, Yuzuru**
**Kawasaki-shi**
**Kanagawa 210-8681 (JP)**
- **AMINO, Yusuke**
**Kawasaki-shi**
**Kanagawa 210-8681 (JP)**

(74) Representative: **Fischer, Heinrich et al**
**Strehl Schübel-Hopf & Partner**
**Maximilianstrasse 54**
**80538 München (DE)**



(56) References cited:
**WO-A1-01/35983**
**WO-A1-2007/055393**
**WO-A2-2006/117211**
**WO-A2-2007/027548**
**WO-A2-2007/055388**
**US-A1- 2003 008 876**
**US-A1- 2005 124 576**
**US-A1- 2007 060 625**

- **GEIBEL J. ET AL.: 'Calcium-sensing receptor abrogates secretagogue-induced increases in intestinal net fluid secretion by enhancing cyclic nucleotide destruction' PROCEEDINGS OF THE NATIONAL ACADEMY OF SCIENCES OF THE USA vol. 103, no. 25, 2006, pages 9390 - 9397, XP001539876**
- **WANG M. ET AL.: 'Activation of Family C G-protein-coupled Receptors by the Tripeptide Glutathione' JOURNAL OF BIOLOGICAL CHEMISTRY vol. 281, no. 13, 31 March 2006, pages 8864 - 8870, XP008123658**


EP 2 156 846 B1


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The present invention relates to a prophylactic or therapeutic agent for diarrhea, which contains, as an active ingredient, a compound having calcium receptor-activating action.

Background Art

The calcium receptor, which is also called the Calcium Sensing Receptor (CaSR)has 1,078 amino acids, and is classified into class C of the seven-transmembrane receptors (G protein-coupled receptor). Cloning of the gene for the calcium receptor was reported in 1993 (Non-patent document 1), and the calcium receptor is known to cause various cell responses via elevation of intracellular calcium levels etc., when activated with calcium etc.. The nucleotide sequence of the human calcium receptor is registered with GenBank Accession No. NM_000388, and is well conserved among animals.

The calcium receptor may act to promote or suppress biological functions. Therefore, at present, therapeutic agent that utilizes an activating action on the calcium receptor and a therapeutic agent that utilizes an inhibitory action on the calcium receptor are appropriately used in the treatment of neurological, hepatic, cardiovascular, digestive diseases, and other diseases, depending on the pathological conditions. For example, the calcium receptor is able to functions to detect increased blood calcium in the parathyroid, and then suppress the secretion of the parathyroid hormone (PTH) to correct the blood calcium level. Therefore, an effect of reducing the blood calcium level is expected for a calcium receptor activator. It has actually been clarified that when the calcium receptor activator is used to treat secondary hyperparathyroidism in a hemodialysis patient, it reduces the PTH level without elevating the calcium and phosphorus levels.

Since a functional analysis of the calcium receptor has been conducted mainly for calcium homeostasis, the applications have so far mainly focused on bone metabolic diseases in which calcium regulation is involved.

However, it has become clear from the results of genetic expression analysis etc., that the calcium receptor is widely distributed in living bodies other than the parathyroid and kidney (Non-patent documents 2 and 3), and the possibility that the calcium receptor is involved in various biological functions and the causes of some diseases has been proposed. For example, there has been speculation that the calcium receptor is involved in the functions of the liver, heart, lung, gastrointestinal tract, lymphocytes, and pancreas. The inventors of the present invention also have confirmed that the calcium receptor is expressed in a wide range of tissues in living bodies by analyzing RNAs extracted from each of rat tissues using RT-PCR. From the above-mentioned viewpoints, the potential applications for activators and inhibitors of the calcium receptor are rapidly increasing.

Moreover, in addition to calcium, cations such as a gadolinium cation, basic peptides such as polyarginine, polyamine such as spermine, amino acids such as phenylalanine, and so forth have been reported as calcium receptor activators (Non-patent document 4).

Non-patent Document 5 reports that glutathione (γ-Glu-Cys-Gly), a low molecular weight peptide, is a calcium receptor activator (that is, has an activating action), but there are no reports of the possibility that glutathione could be effective for the treating diarrhea.

As mentioned above, a number of specific activators have been developed as calcium receptor activators. However, only a few of these compounds are present in the living body, and further, the compounds that are present in the living body have extremely low activities. Therefore, therapeutic drugs for various diseases, that contain these activators, had severe problems in terms of adverse effects, permeability, and activities. For example, although it was known that an amino acid could act on the calcium receptor, the activity of the amino acid was extremely low. Thus, the use of the amino acid to activate the receptor was considered to be difficult. Furthermore, as mentioned above, macromolecules such as polyarginine have been reported as activators, but it is thought that this activity is due to polyvalent cation action of an unspecified structure. In other words, a peptide of a specific structure has not been reported to be useful as a calcium receptor activator.

Diarrhea is a condition that occurs when the moisture present in the stool during defecation is increased, and hence, a loose or liquid stool is excreted. Diarrhea results from the inhibition of moisture absorption due to an intestinal mucosa disorder, the rapid passage of intestine contents due to active peristaltic movement of the intestine, and the activation of intestinal juice secretion from the intestinal mucosa, for example.

Diarrhea is classified, based on its mechanism or cause, into six types: osmotic diarrhea; secretory diarrhea; exudative diarrhea; diarrhea associated with an abnormality in intestinal tract motility; diarrhea due to an abnormality in active transport; and others, and the determination of the mechanism or cause of diarrhea is important in the development of diagnostic and therapeutic strategies.

The current therapy for diarrhea caused by a harmful substance, such as a chemical compound, toxin, or

infectious bacterium, is to administer an adsorbent, such as kaolin-pectin, which can adsorb the harmful substance. Furthermore, the treatment for diarrhea caused by increased gastrointestinal tract motility is to administer a medicament that acts on the central or peripheral nerves and results in suspension of the gastrointestinal tract motility. Still further, when diarrhea is caused by the invasion of harmful bacteria, an antibiotic or an antimicrobial agent can be administered, provided that the bacterium should be specified.

Although therapeutic drugs have been developed depending on the mechanism or cause of the diarrhea thus far, no therapeutic drug based on the electrolyte balance mechanism in the gastrointestinal tract has been reported yet. A therapeutic drug for diarrhea based on the physiological function inherent to the gastro intestinal tract, can be a novel potent therapeutic drug in terms of a function and safety. Therefore, a safe therapeutic drug for diarrhea can be provided.

Non-patent Document 6 mentions the possibility that a calcium receptor activator may serve as a therapeutic drug for diarrhea, but does not disclose whether the calcium receptor actually has a prophylactic or therapeutic effect. The non-patent document also mentions that the calcium receptor activator is desirably non-absorbed in the body from the viewpoint of safety reasons, but does not elucidate the structure of the compound.

Non-patent Document 1: Nature, 1993, Vol. 366(6455), pp. 575-580
Non-patent Document 2: J. Endocrinol., 2000, Vol. 165(2), pp. 173-177
Non-patent Document 3: Eur. J. Pharmacol., 2002, Vol. 447(2-3), pp. 271-278
Non-patent Document 4: Cell Calcium, 2004, Vol. 35(3), pp. 209-216
Non-patent Document 5: J. Biol. Chem., 2006, Vol. 281(13), pp. 8864-8870
Non-patent Document 6: Proc. Natl. Acad. Sci. USA, 2006, Vol. 103(25), pp. 9390-9397

Disclosure of the Invention

Problem to be solved by the Invention

It is as aspect of the present invention is to provide a prophylactic or therapeutic agent for diarrhea, which is highly safe in the living body.

Means for solving the Problem

The inventors of the present invention have researched a calcium receptor activator. As a result, the inventors have found that some peptides have calcium receptor-activating action. The inventors have also found that a compound having calcium receptor-activating action may be a therapeutic drug for diarrhea. On the basis of those findings, the present invention has been completed.

That is, the present invention relates to the following:

[1] An agent comprising a compound having calcium receptor-activating action for use in a method for prophylactic or therapeutic treatment of diarrhea,
wherein the compound is selected from a peptide or a peptide derivative,
wherein the peptide is one kind or two or more kinds selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me)and wherein the peptide derivative has a structure of the following formula (3):

$$\gamma\text{-Glu-X-OCH(Z)}CO_2H \qquad (3)$$

wherein X represents an amino acid or an amino acid derivative and Z represents H or $CH_3$.

[2] The agent according to [1] above, wherein X represents Cys(SNO), Cys(S-allyl), Gly, Cys(S-Me), Cys, Abu, t-Leu, Cle, Aib, Pen or Ser, and Y represents Gly, Val, Glu, Lys, Phe, Ser, Pro, Arg, Asp, Met, Thr, His, Orn, Asn, Cys or Gln.

[3] The agent according to [1] or [2] above, wherein the peptide is γ-Glu-Val-Gly or γ-Glu-t-Leu-Gly.

[4] A compound, which has a structure of the following formula (3):

γ-Glu-X-OCH(Z)$CO_2$H (3)

wherein X represents an amino acid or an amino acid derivative and Z represents $CH_3$; or wherein X represents t-Leu or Abu and Z represents H.

[5] A compound according to [4] above, wherein X represents t-Leu or Abu and Z represents $CH_3$.

[6] A compound according to [4] or [5] above, which is selected from the group consisting of of γ-Glu-Val-GlyA, γ-Glu-t-Leu-GlyA, γ-Glu-Abu-GlyA, γ-Glu-Val-LacA, γ-Glu-t-Leu-LacA, and γ-Glu-Abu-LacA.

[7] A compound, which is represented by γ-Glu-t-Leu-Gly.

Brief Description of the Drawings

FIG. 1 shows a graph illustrating an action of calcium on a calcium receptor. The human calcium receptor cRNA was injected into *Xenopus laevis* oocytes by microinjection. Values of the intracellular response currents were recorded when a calcium chloride solution was added at an arbitrary concentration. The maximum value of the intracellular currents was defined as the response current value (maximum response value). It was confirmed that no response was observed in oocytes injected with distilled water by microinjection as a control.

FIG. 2 shows a graph illustrating an action of an L-amino acid on a calcium receptor. The human calcium receptor cRNA was injected into *Xenopus laevis* oocytes by microinjection. Values of the intracellular response currents were recorded when a 10 mM L-amino acid solution was added. The maximum value of the intracellular currents was defined as the response current value. It was confirmed that no response was observed in oocytes injected with distilled water by microinjection as a control.

FIG. 3 shows a graph illustrating an action of a D-amino acid on a calcium receptor. The human calcium receptor cRNA was injected into *Xenopus laevis* oocytes by microinjection. Values of the intracellular response currents were recorded when a 10 mM D-amino acid solution was added. The maximum value of the intracellular currents was defined as the response current value. It was confirmed that no response was observed in oocytes injected with distilled water by microinjection as a control.

FIG. 4 shows a graph illustrating an action of a peptide on a calcium receptor. The human calcium receptor cRNA was injected into *Xenopus laevis* oocytes by microinjection. Values of intracellular response currents were recorded when a peptide solution was added at an arbitrary concentration. The maximum value of the intracellular currents was defined as the response current value. It was confirmed that no response was observed in oocytes injected with distilled water by microinjection as a control.

FIG. 5 shows a graph illustrating a therapeutic effect on diarrhea of a peptide having calcium receptor-activating action. In mice 5-HTP-induced defecation model, 0.1 and 1% of the peptide γEVG having the calcium receptor-activating action each improved a stool form score in a dose-dependent manner.

FIG. 6 shows a graph illustrating the effect of γEVG on fluid absorption in a large intestine loop method.

FIG. 7 shows a graph illustrating the effects of GSH and γ-Glu-t-Leu-Gly on fluid absorption in a large intestine loop method.

FIG. 8 shows a graph illustrating the effect of cinacalcet on fluid absorption in a large intestine loop method.

Best Mode for carrying out the Invention

Hereinafter, the present invention is described in detail.

A prophylactic or therapeutic agent for diarrhea of the present invention contains a compound having calcium receptor-activating action.

The term "calcium receptor" in the present description means a receptor that is called the Calcium Sensing Receptor (CaSR) and belongs to class C of the seven-transmembrane receptors. The term "calcium receptor activator" in the present description means a substance that binds to the above-mentioned calcium receptor to activate the calcium receptor. Further, the phrase "to activate a calcium receptor" in the present description means that a ligand binds to a calcium receptor to activate a guanine nucleotide binding protein, to thereby transmit a signal. In addition, the term "calcium receptor activity" means that the calcium receptor transmits a signal.

<1> Compound having calcium receptor-activating action

The compound having calcium receptor-activating action is a peptide or a derivative thereof. The compound having a calcium receptor-activating action may also be obtained by reacting a calcium receptor with a test substance; and detecting calcium receptor activity. It is preferably confirmed that the thus obtained peptide has a prophylactic or therapeutic effect on diarrhea.

Hereinafter, a method of screening the compound having a calcium receptor-activating action is specifically described:

1) measuring a calcium receptor activity by adding a test substance to a measurement system of calcium receptor activity for measuring the calcium receptor activity;
2) comparing a calcium receptor activity when adding the test substance with a calcium receptor activity when being free of the test substance; and
3) selecting the test substance exhibiting a high calcium receptor-stimulating activity when the test substance is added.

The calcium receptor activity is, for example, measured by using a measurement system using cells that express calcium receptors. The above-mentioned cells may be cells including endogenously expressed calcium receptors, or may be recombinant cells in which exogenous calcium receptor genes are introduced. The measurement system of calcium receptor activity described above may be used without any particular limitation as long as, when an extracellular ligand (activator) specific to a calcium receptor is added to the above-mentioned cells that express calcium receptors, the measurement system may detect the binding (reaction) between the activator and the calcium receptor, or may respond to the binding (reaction) between the activator and the calcium receptor to thereby transmit a detectable signal into the cells. When the calcium receptor activity is detected through the reaction with the test substance, it is determined that the test substance has a calcium receptor-stimulating activity, and is a substance having a prophylactic or therapeutic effect on diarrhea.

Meanwhile, the prophylactic or therapeutic effect on diarrhea may be confirmed by a test using an anticancer agent-induced diarrhea model described in examples, mice 5-HTP-induced defecation model. Furthermore, the compounds to be used as a test substance are not particularly limited. However, the peptide is preferably a peptide consisting of 2 to 10 amino acid residues, or a derivative thereof, and more preferably a peptide consisting of 2 or 3 amino acid residues or a derivative thereof. The amino acid residue at the N-terminal side of the peptide is preferably $\gamma$-glutamic acid.

The origin of the calcium receptor is not particularly limited. Examples thereof include not only the human calcium receptor, but also a calcium receptor derived from an animal such as a mouse, a rat, and a dog. Specifically, preferred examples of the above-mentioned calcium receptor may include the human calcium receptor encoded by the human calcium receptor gene registered with GenBank Accession No NM_000388. The calcium receptor is not limited to the protein encoded by the gene having the above-mentioned sequence, and may be a protein encoded by a gene having a 60% or more, preferably 80% or more, and more preferably 90% or more homology to the above-mentioned sequence as long as the gene encodes a protein having a calcium receptor function. The GPRC6A receptor or 5.24 receptor is also known as a subtype of the calcium receptor, and can be used in the present invention. It should be noted that the calcium receptor function may be investigated by expressing the genes in cells , and measuring the change in the current when calcium is added, and the change in the intracellular calcium ion concentration.

As described above, the calcium receptor activity can be confirmed by using live cells expressing a calcium receptor or its fragment, cell membranes expressing a calcium receptor or its fragment, an in vitro system containing a protein of a calcium receptor or its fragment.

An example using live cells is described below. However, confirmation of the calcium receptor activity is not limited to this example.

A calcium receptor is expressed in cultured cells such as *Xenopus laevis* oocytes, hamster ovarian cells, and human fetal kidney cells. The calcium receptor can be expressed by cloning the calcium receptor gene in a plasmid that carries a foreign gene and introducing the plasmid or cRNA obtained by using the plasmid as a template. To detect the reaction, an electrophysiological technique and a fluorescent indicator that indicates an increase in intracellular calcium level can be used.

Expression of the calcium receptor is first confirmed based on the response to calcium or a specific activator. Oocytes that showed intracellular current with calcium at a concentration of about 5 mM, or cultured cells that showed fluorescence of the fluorescent indicator reagent with calcium at a concentration of about 5 mM are used. The calcium concentration dependency is determined by changing the calcium concentration. Then, a test substance such as a peptide is prepared to a concentration of about 1 $\mu$M to 1 mM, and added to the oocytes or cultured cells, and the calcium receptor activity of the above-mentioned peptide is determined.

The compounds having calcium receptor-activating action to be used in the present invention are peptides or

derivatives thereof (Hereinafter, when "peptide" is simply used, the peptide sometimes means both of a peptide and a peptide derivative). The peptide is γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me) (hereinafter, also referred to as a "peptide of the present invention" together with the peptide derivative described below).

Further, the peptide may be a peptide derivative having a structure of γ-Glu-X-OCH(Z)$CO_2$H where X represents an amino acid or an amino acid derivative, and Z represents H (a hydrogen atom) or $CH_3$ (a methyl group). Suitable specific examples thereof include γ-Glu-Val-GlyA, γ-Glu-t-Leu-GlyA, γ-Glu-Abu-GlyA, γ-Glu-Val-LacA, γ-Glu-t-Leu-LacA, and γ-Glu-Abu-LacA. It should be noted that GlyA represents glycolic acid and LacA represents lactic acid. Lactic acid may be one of S-lactic acid and R-lactic acid, and preferably is S-lactic acid. Structural formulae of these compounds are described below.

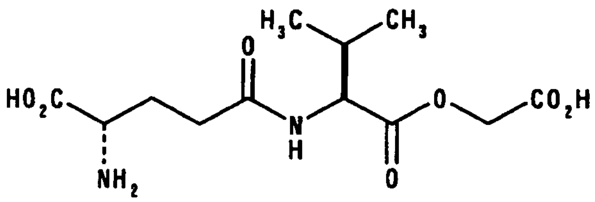

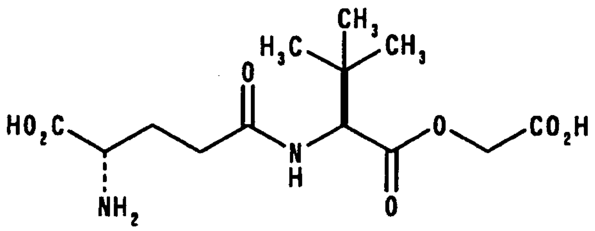

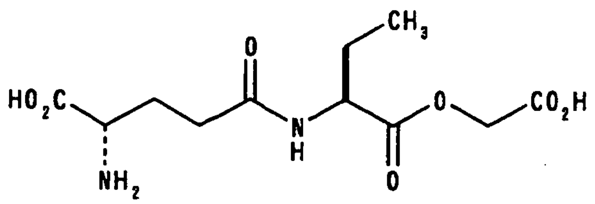

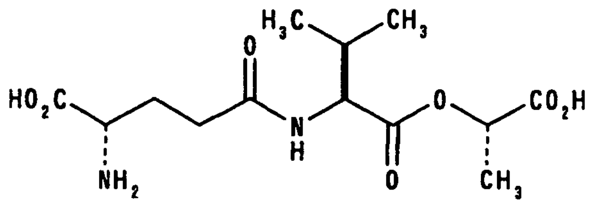

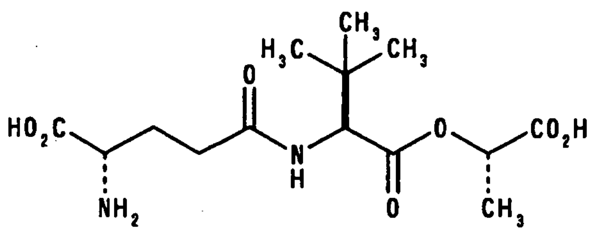

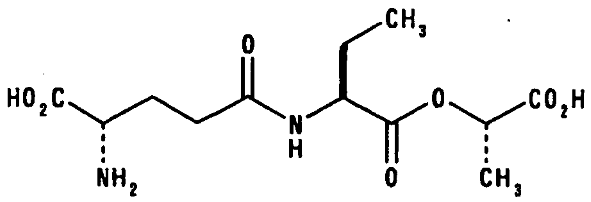

Of the compounds γ-Glu-X-Gly where X represents an amino acid or an amino acid derivative and γ-Glu-Val-Y where Y represents an amino acid or an amino acid derivative, preferred compounds are compounds in which X represents Cys(SNO), Cys(S-allyl), Gly, Cys(S-Me), Cys, Abu, t-Leu, Cle, Aib, Pen, or Ser, and Y represents Gly, Val, Glu, Lys, Phe, Ser, Pro, Arg, Asp, Met, Thr, His, Orn, Asn, Cys, Gln, GlyA, or LacA. Of those, particularly preferred compounds are γ-Glu-Val-Gly and γ-Glu-t-Leu-Gly.

It should be noted that herein, an amino acid of which each peptide consists is an L-amino acid unless otherwise stated. Herein, examples of the amino acid include: a neutral amino acid such as Gly, Ala, Val, Leu, Ile, Ser, Thr, Cys, Met, Asn, Gln, Pro, and Hyp, an acidic amino acid such as Asp and Glu; a basic amino acid such as Lys, Arg, and His; an aromatic amino acid such as Phe, Tyr, and Trp; and homoserine, citrulline, ornithine, α-aminobutyric acid, norvaline, norleucine, and taurine. The amino acid may also be a non-naturally occurring (non-protein constituent) amino acid such as tert-leucine, cycloleucine, α-aminoisobutyric acid, and L-penicillamine. It should be noted that X in the peptide γ-Glu-X-Gly may be any one of such an amino acid or a derivative thereof described above, and preferably an amino acid or a derivative thereof, other than Cys.

Herein, abbreviations for amino residues mean the following amino acids.

(1) Gly: Glycine
(2) Ala: Alanine
(3) Val: Valine
(4) Leu: Leucine
(5) Ile: Isoleucine
(6) Met: Methionine
(7) Phe: Phenylalanine
(8) Tyr: Tyrosine
(9) Trp: Tryptophan
(10) His: Histidine
(11) Lys: Lysine
(12) Arg: Arginine
(13) Ser: Serine
(14) Thr: Threonine
(15) Asp: Aspartic acid
(16) Glu: Glutamic acid
(17) Asn: Asparagine
(18) Gln: Glutamine
(19) Cys: Cysteine
(20) Pro: Proline
(21) Orn: Ornithine
(22) Sar: Sarcosine
(23) Cit: Citrulline
(24) N-Val: Norvaline
(25) N-Leu: Norleucine
(26) Abu: α-Aminobutyric acid
(27) Tau: Taurine
(28) Hyp: Hydroxyproline
(29) t-Leu: tert-Leucine
(30) Cle: Cycloleucine
(31) Aib: α-Aminoisobutyric acid (2-methylalanine)
(32) Pen: L-Penicillamine

Examples of the amino acid derivatives include various derivatives of the above amino acids such as an unusual amino acid, a non-natural amino acid, an amino alcohol, and a substituted amino acid with a side chain such as the

terminal carbonyl group, the terminal amino group, and the thiol group of cysteine, that can contain various substituents. Examples of the substituent include an alkyl group, an acyl group, a hydroxy group, an amino group, an alkylamino group, a nitro group, a sulfonyl group, and various protection groups. Examples of the substituted amino acid include: Arg($NO_2$):N-y-nitroarginine;Cys(SNO):S-nitrocysteine;Cys(S-Me): S-methylcysteine; Cys(S-allyl): S-allylcysteine; Val-$NH_2$: valinamide; and Val-ol: valinol (2-amino-3-methyl-1-butanol).

It should be noted that γ-Glu-Cys(SNO)-Gly described above has the following structural formula, and the "(O)" in the above formulae γ-Glu-Met(O) and γ-Glu-Cys(S-Me)(O) indicates a sulfoxide structure.

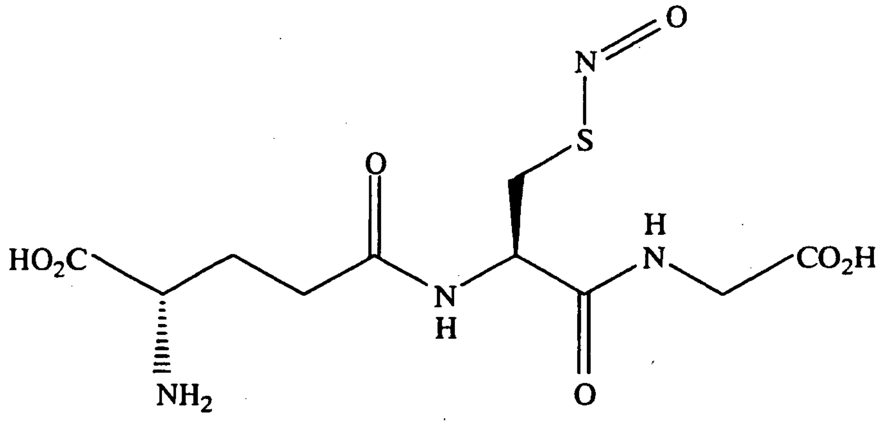

S-Nitrosoglutathione(GNSO)

The inventors of the present invention have revealed that γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me) each activate the calcium receptor. Therefore, γ-Glu-X-Gly where X represents an amino acid or an amino acid derivative, γ-Glu-Val-Y where Y represents an amino acid or an amino acid derivative, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me) can be used as a therapeutic agent for diarrhea. The peptide to be used in the present invention may be used alone or may be used as a mixture of arbitrary two kinds or three or more kinds in the present invention.

As the above-mentioned peptide, a commercially-available product can be used. Furthermore, the peptide can be obtained by appropriately using a known technique such as (1) a method of chemically synthesis, or (2) a method of synthesizing the peptide by an enzymatic reaction. Since the number of amino acid residues contained in the peptide to be used in the present invention is as comparatively small as 2 or 3 residues, a method of chemically synthesis is convenient. When chemically synthesizing the peptide, the oligopeptide can be synthesized or semi-synthesized by using a peptide synthesizer. An example of the method of chemically synthesis of the peptide includes a peptide solid phase synthetic method. The peptide synthesized as described above can be purified by usual means such as ion exchange chromatography, reversed phase high performance liquid chromatography, or affinity chromatography. Such a solid phase synthetic method of the peptide and the subsequent peptide purification are well known in the technical field.

Further, the peptide to be used in the present invention can also be produced by an enzymatic reaction. For example, the method described in WO 2004/011653 can be used. That is, the peptide can also be produced by: reacting one amino acid or dipeptide having an esterified or amidated carboxyl terminus with an amino acid having a free amino group (for example, an amino acid with a protected carboxyl group) in the presence of a peptide producing enzyme; and purifying the produced dipeptide or tripeptide. Examples of the peptide-producing enzyme include a culture of a microorganism having the ability to produce the peptide, microbial cells separated from the culture, or a processed product of cells of the microorganism, or a peptide producing enzyme derived from the microorganism.

The compound to be used in the present invention also include that in the form of a salt. When the peptide of the present invention is in the form of a salt, the salt may be a pharmacologically acceptable salt. Examples of a salt with an acidic group such as a carboxyl group in the formula include: an ammonium salt; a salt with an alkali metal such as sodium and potassium; a salt with an alkaline earth metal such as calcium and magnesium, an aluminum salt, a zinc salt; a salt with an organic amine such as triethylamine, ethanolamine, morpholine, pyrrolidine, piperidine, piperazine, and dicyclohexylamine; and a salt with a basic amino acid such as arginine and lysine. Examples of a salt with a basic

group in case where the basic group exists in the formula include: a salt with an inorganic acid such as hydrochloric acid, sulfuric acid, phosphoric acid, nitric acid, and hydrobromic acid; a salt with an organic carboxylic acid such as acetic acid, citric acid, benzoic acid, maleic acid, fumaric acid, tartaric acid, succinic acid, tannic acid, butyric acid, hibenzoic acid, pamoic acid, enanthoic acid, decanoic acid, teoclic acid, salicylic acid, lactic acid, oxalic acid, mandelic acid, and malic acid; and a salt with an organic sulfonic acid such as methanesulfonic acid, benzenesulfonic acid, and p-toluenesulfonic acid.

<2> Prophylactic or therapeutic agent for diarrhea

The compound having calcium receptor-activating action may be used as an active ingredient in a prophylactic or therapeutic agent for diarrhea. Examples of the form of the prophylactic or therapeutic agent for diarrhea of the present invention include pharmaceuticals, quasi drugs, and foods.

A method of administering the prophylactic or therapeutic agent for diarrhea is not particularly limited, and can include oral administration, an invasive administration utilizing an injection, a suppository administration, or a transdermal administration. The prophylactic or therapeutic agent for diarrhea of the present invention may be administered in the form of a conventionally-used pharmaceutical formulation by mixing its active ingredient with a nontoxic solid or liquid pharmaceutical carrier, which is suitable for oral or injectable administration. Examples of these formulations include: a form of a solid formulation such as a tablet, a granule, a powder, and a capsule; a form of a liquid formulation such as a solution, a suspension, and an emulsion; and a form of a lyophilizate. These formulations may be prepared by practices for preparing the formulations.

Examples of the above-mentioned nontoxic carriers for pharmaceuticals include glucose, lactose, sucrose, starch, mannitol, dextrin, fatty acid glyceride, polyethylene glycol, hydroxyethyl starch, ethylene glycol, polyoxyethylene sorbitan fatty acid ester, gelatin, albumin, amino acid, water, and physiological saline. Further, if required, a conventionally-used additive agent such as a stabilizing agent, a wetting agent, an emulsifier, a binder, and a tonicity agent may be appropriately added.

The compound having calcium receptor-activating action to be used for the prophylactic or therapeutic agent for diarrhea of the present invention is preferably a peptide of the present invention or a lowmolecular weight compound of the present invention, and may be a known compound having calcium receptor-activating action. Furthermore, the prophylactic or therapeutic agent for diarrhea of the present invention may contain, in addition to the peptide of the present invention, one kind or two or more kinds of known calcium receptor activators.

Examples of the above-mentioned known calcium receptor activators include a cation such as a calcium cation and a gadolinium cation; a basic peptide such as polyarginine and polylysine; a polyamine such as putrescine, spermine, and spermidine; a protein such as protamine; an amino acid such as phenylalanine; a peptide such as glutathione; and an analogous compound as cinacalcet. In the present invention, the above-mentioned known calcium receptor activator may be added alone or may be added as a mixture of any two kinds or three or more kinds thereof. Of the above-mentioned known calcium receptor activators, preferred is a cation such as a calcium cation and a gadolinium cation, and more preferred is a calcium cation. In other words, at least one kind of the known calcium receptor activator to be further added is preferably a cation.

When a known calcium receptor activator is mixed with the peptide of the present invention as described herein, stronger activation of the calcium receptor is observed. When the peptide of the present invention is used as the calcium receptor activator, the ratio of the total of the peptide of the present invention to the total of a known calcium receptor activator is not particularly limited as long as stronger activation on the calcium receptor is achieved. For example, the mass ratio of the total of the known calcium receptor activator to the total of the peptide of the present invention is preferably set to 1:100 to 100:1.

The administration or intake amount of the prophylactic or therapeutic agent for diarrhea of the present invention may be any as long as the amount is effective for therapy or prophylaxis, and is appropriately adjusted depending on the age, sex, body weight, and symptom of the patient. For example, in the case of an oral administration, the total amount of the peptide to be used in the present invention is preferably 0.01 g to 10 g per kg body weight per dose and more preferably 0.1 g to 1 g per kg body weight per dose. The administration frequency is not particularly limited, and the administration may be performed once to several times per day.

The amount of the compound having calcium receptor-activating action in the prophylactic or therapeutic agent for diarrhea of the present invention is not limited as long as the amount is suited to the above-described dosage. The amount is preferably 0.000001% by mass to 99.9999% by mass, more preferably 0.00001% by mass to 99.999% by mass, and particularly preferably 0.0001% by mass to 99.99% by mass with respect to the dry weight.

The prophylactic or therapeutic agent for diarrhea of the present invention may also be used as a food or drink having an effect for therapy or prophylaxis of diarrhea. For example, the prophylactic or therapeutic agent may be formulated into a food or drink in a container or packaging which indicates that the agent has a therapeutic or prophylaxis effect for diarrhea. The form of the food or drink is not particularly limited, and the food or drink may be produced in the

same production method as a general food and with the same materials as those for the general food except that the compound having calcium receptor-activating action is blended. Examples of the food include a seasoning, a drink such as juice and cow milk, a confectionery, a jelly, a health food, a processed agricultural product, a processed fishery product, a processed animal product such as cow milk, and a food supplement. Further, examples of diarrhea in the present invention include irritable bowel syndrome, functional diarrhea, inflammatory bowel disease, tympanitis, bacterial diarrhea, and dyspepsia.

Examples

Hereinafter, the present invention is more specifically described with reference to examples.

[Example 1] Preparation of a calcium receptor gene (cRNA)

The gene encoding the calcium receptor was prepared as follows. On the basis of the DNA sequence registered at NCBI (calcium receptor: NM_000388), synthetic oligo DNAs (forward primer (SEQ ID NO: 1) and reverse primer (SEQ ID NO: 2)) used for PCR were synthesized.

Human kidney cDNA (manufactured by Clontech) was used as a source, and PCR was performed by using the primers and Pfu ultra DNA Polymerase (manufactured by Stratagene) under the following conditions. After a reaction at 94°C for 3 minutes, a cycle of reactions at 94°C for 30 seconds, 55°C for 30 seconds, and 72°C for 2 minutes was repeated 35 times, and then a reaction was performed at 72°C for 7 minutes. Whether amplification was attained by PCR was detected by performing agarose electrophoresis, staining with a DNA staining reagent, and subsequent ultraviolet irradiation. The chain lengths of the PCR products were confirmed by comparison with DNA markers of known sizes which were simultaneously subjected to the electrophoresis. The plasmid vector pBR322 was digested with the restriction enzyme EcoRV (manufactured by Takara). The gene fragment amplified by PCR was ligated to the cleavage site of the plasmid by using Ligation Kit (manufactured by Promega). The *Escherichia coli* DH5α strain was transformed with each ligation reaction solution, and a transformant harboring the plasmid in which the PCR amplification product was cloned was selected. The PCR amplification product was confirmed by DNA sequence analysis. By using the recombinant plasmid as a template, cRNA of the calcium receptor gene was prepared using a cRNA preparation kit (manufactured by Ambion).

[Example 2] Preparation of various samples

As L-amino acid samples, 23 kinds of special grade amino acids including alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, ornithine, and taurine (all from Ajinomoto Co., Inc.), and hydroxyproline (Nacarai Tesque, Inc.), were used. For D-Cys and D-Trp (Nacarai Tesque, Inc.) and calcium chloride, a special grade was used.

Furthermore, as peptide specimens, γ-Glu-Cys-Gly (Sigma Aldrich Japan K.K.), γ-Glu-Cys(SNO)-Gly (Dojindo Laboratories), γ-Glu-Ala (Bachem Feinchemikalien AG), γ-Glu-Gly (Bachem Feinchemikalien AG), γ-Glu-Cys (Sigma Aldrich Japan K.K.), γ-Glu-Met (Bachem Feinchemikalien AG), γ-Glu-Abu-Gly (Abu: α-aminobutyric acid, Bachem Feinchemikalien AG), γ-Glu-Thr (Kokusan Chemical Co., Ltd.), γ-Glu-Val (Kokusan Chemical Co., Ltd.), γ-Glu-Leu (custom synthesis product), y-Glu-Ile (custom synthesis product), γ-Glu-Orn (Kokusan Chemical Co., Ltd.), Asp-Gly (custom synthesis product), Cys-Gly (custom synthesis product), Cys-Met (custom synthesis product), Glu-Cys (custom synthesis product), Gly-Cys (custom synthesis product), Leu-Asp (custom synthesis product), γ-Glu-Val-Val (custom synthesis product), γ-Glu-Val-Glu (custom synthesis product), γ-Glu-Val-Lys (custom synthesis product), γ-Glu-γ-Glu-Val (custom synthesis product), γ-Glu-Gly-Gly (custom synthesis product), γ-Glu-Val-Phe (custom synthesis product), γ-Glu-Val-Ser (custom synthesis product), γ-Glu-Val-Pro (custom synthesis product), γ-Glu-Val-Arg (custom synthesis product), γ-Glu-Val-Asp (custom synthesis product), γ-Glu-Val-Met (custom synthesis product), γ-Glu-Val-Thr (custom synthesis product), γ-Glu-Val-His (custom synthesis product), γ-Glu-Val-Asn (custom synthesis product), γ-Glu-Val-Gln (custom synthesis product), γ-Glu-Val-Cys (custom synthesis product), γ-Glu-Val-Orn (custom synthesis product), γ-Glu-Ser-Gly (custom synthesis product), and γ-Glu-Pen-Gly (custom synthesis product) were used. Glutamine and cysteine were prepared upon use, and the other samples were stored at -20°C after preparation. Peptides having a purity of 90% or higher were used, except for γ-Glu-Cys, which was 80% or higher.

After dissolving each sample in solution, if the pH of the solution is either acidic or alkaline, the pH of the solution was adjusted to an approximately neutral pH by using NaOH or HCl. The solution used for dissolution of amino acids and peptides, preparation of *Xenopus laevis* oocytes, and culture of the oocytes had the following composition: 96 mM NaCl, 2 mM KCl 1 mM $MgCl_2$, 1.8 mM $CaCl_2$, 5 mM Hepes, pH 7.2.

[Example 3] Synthesis of γ-Glu-Val-Gly

Boc-Val-OH (8.69 g, 40.0 mmol) and Gly-OBzl·HCl (8.07 g, 40.0 mmol) were dissolved in methylene chloride (100 ml) and the solution was kept at 0°C. Triethylamine (6.13 ml, 44.0 mmol), HOBt (1-hydroxybenzotriazole, 6.74 g, 44.0 mmol), and WSC·HCl (1-ethyl-3-(3-dimethylaminopropyl)carbodiimide hydrochloride, 8.44 g, 44.0 mmol) were added to the solution, and the mixture was stirred overnight at room temperature. The reaction solution was concentrated under reduced pressure, and the residue was dissolved in ethyl acetate (200 ml). The solution was washed with water (50 ml), a 5% citric acid aqueous solution (50 ml × twice), saturated brine (50 ml), a 5% sodium bicarbonate aqueous solution (50 ml × twice), and saturated brine again (50 ml). The organic layer was dried over anhydrous magnesium sulfate, magnesium sulfate was removed by filtration, and the filtrate was concentrated under reduced pressure. The residue was recrystallized from ethyl acetate/n-hexane to obtain Boc-Val-Gly-OBzl (13.2 g, 36.2 mmol) as a white crystal.
Boc-Val-Gly-OBzl (5.47 g, 15.0 mmol) was added to a 4 N HCl/dioxane solution (40 ml), and the mixture was stirred at room temperature for 50 minutes. Dioxane was removed by concentration under reduced pressure, n-hexane (30 ml) was added to the residue, and the mixture was concentrated under reduced pressure. The procedure was repeated 3 times to quantitatively obtain H-Val-Gly-OBzl·HCl.
H-Val-Gly-OBzl·HCl and Z-Glu-OBzl (5.57 g, 15.0 mmol) described above were dissolved in methylene chloride (50 ml), and the solution was maintained at 0°C. Triethylamine (2.30 ml, 16.5 mmol), HOBt (1-hydroxybenzotriazole, 2.53g, 16.5mmol), and WSC·HCl (1-ethyl-3-(3-dimethylaminopropyl)carbodiimide hydrochloride, 3.16 g, 16.5 mmol) were added to the solution, and the mixture was stirred at room temperature overnight for 2 days. The reaction solution was concentrated under reduced pressure, and the residue was dissolved in heated ethyl acetate (1,500 ml). The solution was washed with water (200 ml), 5% citric acid aqueous solution (200 ml × twice), saturated brine (150 ml), 5% sodium bicarbonate aqueous solution (200 ml × twice), and saturated brine again (150 ml). The organic layer was dried over anhydrous magnesium sulfate, magnesium sulfate was removed by filtration, and the filtrate was concentrated under reduced pressure. The precipitated crystal was collected by filtration and dried under reduced pressure to obtain Z-Glu(Val-Gly-OBzl)-OBzl (6.51 g, 10.5 mmol) as a white crystal.
Z-Glu(Val-Gly-OBzl)-OBzl described above (6.20 g, 10.03 mmol) was suspended in ethanol (200 ml), 10% palladium/carbon (1.50 g) was added to the suspension , and a reduction reaction was performed under a hydrogen atmosphere at 55° C for 5 hours. During the reaction, 100 ml in a total volume of water was gradually added. The catalyst was removed by filtration using a Kiriyama funnel (Kiriyama glass Co.), and the filtrate was concentrated under reduced pressure to a half volume. The reaction solution was further filtered through a membrane filter, and the filtrate was concentrated under reduced pressure. After the residue was dissolved in a small volume of water, ethanol was added to precipitate a crystal, and the crystal was collected by filtration and dried under reduced pressure to obtain γ-Glu-Val-Gly as a white powder (2.85 g, 9.40 mmol).
ESI-MS: $(M+H)^+$=304.1.
$^1$H-NMR (400 MHz, $D_2O$) δ (ppm): 0.87 (3H, d, J=6.8 Hz), 0.88 (3H, d, J=6.8 Hz), 1.99-2.09 (3H, m), 2.38-2.51 (2H, m) 3.72 (1H, t, J=6.35 Hz), 3.86 (1H, d, J=17.8 Hz), 3.80 (1H, d, J=17.8 Hz), 4.07 (1H, d, J=6.8 Hz).

[Example 4] Synthesis of γ-Glu-Cys(S-Me)-Gly [Cys(S-Me): S-methylcysteine]

Reduced glutathione (15.0 g, 48.8 mmol) was added to water (45 ml) and sodium hydroxide (4.52 g, 2.2 equivalents, 107 mmol) was added portionwise to the mixture under bubbling with nitrogen. Methyl iodide (4.56 ml, 1.5 equivalents, 73 mmol) was added to the mixture, and the solution was sealed and stirred at room temperature for 2 hours. The reaction solution was adjusted to pH 2 to 3 with concentrated hydrochloric acid, supplemented with ethanol (150 ml), and stored overnight in a refrigerator. Since an oily product was separated, the supernatant was removed. When the remaining oily product was dissolved in water and gradually supplemented with ethanol, a partially crystallized oily product precipitated. Therefore, the supernatant was removed again. The residue was dissolved in water (300 ml), adsorbed to a column filled with an ion exchange resin (Dowex 1-acetate, 400 ml), washed with water, and then eluted with a 1 N acetic acid aqueous solution. The eluate was concentrated under reduced pressure, and reprecipitated from water/ethanol to obtain γ-Glu-Cys(S-Me)-Gly as a white powder (5.08 g, 15.8 mmol).
FAB-MS: $(M+H)^+$=322.
$^1$H-NMR (400 MHz, $D_2O$) δ (ppm): 2.14 (3H, s), 2.15-2.22 (2H, m), 2.50-2.58 (2H, m), 2.86 (1H, dd, J=9.0 Hz, J=14.0 Hz), 3.03 (1H, dd, J=5.0 Hz, J=14.0 Hz), 3.84 (1H, t, J=6.5 Hz), 3.99 (2H, s), 4.59 (1H, dd, J=5.0 Hz, J=9.0 Hz)

[Example 5] Synthesis of other peptides

γ-Glu-Met(O), γ-Glu-Val-$NH_2$, γ-Glu-Val-ol, γ-Glu-Ser, γ-Glu-Tau, γ-Glu-Cys(S-Me)(O), γ-Glu-t-Leu, γ-Glu-Cys(S-allyl)-Gly, γ-Glu-Cys(S-Me), γ-Glu-Cle-Gly, and γ-Glu-Aib-Gly were synthesized in accordance with Examples 3 and 4.

[Example 6] Synthesis of γ-Glu-t-Leu-Gly

Boc-t-Leu-OH (9.26 g, 40.0 mmol) and Gly-OBzl·HCl (8.06 g, 40.0 mmol) were dissolved in methylene chloride (60 ml) and the solution was kept at 0°C. Triethylamine (5.60 ml, 40.0 mmol), HOBt (1-hydroxybenzotriazole, 6.75 g, 44.0 mmol), and WSC·HCl (1-ethyl-3-(3-dimethylaminopropyl)carbodiimide hydrochloride, 8.47 g, 44.0 mmol) were added to the solution, and the mixture was stirred overnight at room temperature. The reaction solution was concentrated under reduced pressure, and the residue was dissolved in ethyl acetate (200 ml). The solution was washed with water (50 ml), 5% citric acid aqueous solution (50 ml × twice), saturated brine (50 ml), 5% sodium bicarbonate aqueous solution (50 ml × twice), and saturated brine again (50 ml). The organic layer was dried over anhydrous magnesium sulfate, magnesium sulfate was removed by filtration, and the filtrate was concentrated under reduced pressure. The residue was recrystallized from ethyl acetate/n-hexane to obtain Boc-t-Leu-Gly-OBzl (15.20 g, 40.1 mmol) as a viscous, oily product.

Boc-t-Leu-Gly-OBzl (15.20 g, 40.1 mmol) was added to a 4 N HCl/dioxane solution (200 ml), and the mixture was stirred at room temperature for 1 hour. Dioxane was removed by concentration under reduced pressure, n-hexane (30 ml) was added to the residue, and the mixture was concentrated under reduced pressure. The procedure was repeated 3 times to quantitatively obtain H-t-Leu-Gly-OBzl·HCl

H-t-Leu-Gly-OBzl·HCl and Z-Glu-OBzl (14.93 g, 40.2 mmol) described above were dissolved in methylene chloride (80 ml), and the solution was kept at 0° C. Triethylamine (5.60 ml, 40.2 mmol), HOBt (6.79 g, 44.2 mmol), and WSC·HCl (8.48 g, 44.2 mmol) were added to the solution, and the mixture was stirred at room temperature overnight for 2 days. The reaction solution was concentrated under reduced pressure, and the residue was dissolved in heated ethyl acetate (300 ml). The solution was washed with water (100 ml), 5% citric acid aqueous solution (100 ml × twice), saturated brine (100 ml), 5% sodium bicarbonate aqueous solution (100 ml × twice), and saturated brine again (100 ml). The organic layer was dried over anhydrous magnesium sulfate, magnesium sulfate was removed by filtration, and the filtrate was concentrated under reduced pressure. The residue was purified by silica gel chromatography to obtain Z-Glu(t-Leu-Gly-OBzl)-OBzl (16.10 g, 25.5 mmol) as a viscous, oily product.

Z-Glu(t-Leu-Gly-OBzl)-OBzl described above (16.10 g, 25.5 mmol) was suspended in ethanol (300 ml), 10% palladium carbon (2.00 g) was added to the suspension , and a reduction reaction was performed under a hydrogen atmosphere at room temperature for 5 hours. During the reaction, 100 ml in a total volume of water were gradually added. The catalyst was removed by filtration using a Kiriyama funnel (Kiriyama glass Co.), and the filtrate was concentrated under reduced pressure to a half volume. The reaction solution was further filtered through a membrane filter, and the filtrate was concentrated under reduced pressure. After the residue was dissolved in a small volume of water, ethanol was added to precipitate a crystal, and the crystal was collected by filtration and freeze dried to obtain γ-Glu-t-Leu-Gly as a white powder (6.70 g, 21.1 mmol).

ESI-MS: $(M+H)^+$=318.10.

$^1$H-NMR (400 MHz, $D_2O$) δ (ppm): 0.95 (9H, s), 2.04-2.08 (2H, m), 2.45-2.48 (2H, m), 3.73 (1H, t), 3.87-3.90 (2H, m), 4.07 (1H, s).

[Example 7] Synthesis of γ-Glu-Abu-GlyA

Boc-Abu-OH (6.10 g, 30.0 mmol) and benzyl glycolate (H-GlyA-OBzl, 4.39g, 30.0 mmol) were dissolved in methylene chloride (40 ml) and the solution was kept at 0°C. DMAP (4-dimethylaminopyridine, 1.10 g, 9.0 mmol) and WSC·HCl (6.33 g, 33.0 mmol) were added to the solution, and the mixture was stirred at room temperature overnight. The reaction solution was concentrated under reduced pressure, and the residue was dissolved in ethyl acetate (150 ml). The solution was washed with water (50 ml), 5% citric acid aqueous solution (50 ml × twice), saturated brine (50 ml) , 5% sodium bicarbonate aqueous solution (50 ml × twice), and saturated brine again (50 ml). The organic layer was dried over anhydrous magnesium sulfate, magnesium sulfate was removed by filtration, and the filtrate was concentrated under reduced pressure to obtain Boc-Abu-GlyA-OBzl (9.47 g, 28.1 mmol) as a viscous, oily product.

To the above-mentioned residue, a 4 N HCl/dioxane solution (141 ml) was added, and the mixture was stirred at room temperature for 1 hour. Dioxane was removed by concentration under reduced pressure, n-Hexane (30 ml) was added to the residue, and the mixture was concentrated under reduced pressure. The procedure was repeated 3 times to quantitatively obtain H-Abu-GlyA-OBzl·HCl.

H-Abu-GlyA-OBzl·HCl and Z-Glu-OBzl (10.47 g, 28.1 mmol) described above were dissolved in methylene chloride (100 ml) and the solution was kept at 0° C. Triethylamine (4.30 ml, 30.9 mmol), HOBt (4.74 g, 30.9 mmol), and WSC·HCl (5.95 g, 30.9 mmol) were added to the solution, and the mixture was stirred at room temperature overnight for 2 days. The reaction solution was concentrated under reduced pressure, and the residue was dissolved in ethyl acetate (200 ml). The solution was washed with water (50 ml), 5% citric acid aqueous solution (150 ml × twice), saturated brine (50 ml), 5% sodium bicarbonate aqueous solution (50 ml × twice), and saturated brine again (50 ml). The organic layer was dried over anhydrous magnesium sulfate, magnesium sulfate was removed by filtration, and the filtrate was

concentrated under reduced pressure. The residue was purified by silica gel chromatography to obtain Z-Glu(Abu-GlyA-OBzl)-OBzl (11.20 g, 19.0 mmol) as a viscous, oily product.

Z-Glu(Abu-GlyA-OBzl)-OBzl (11.20 g, 19.0 mmol) described above was suspended in ethanol (150 ml), 10% palladium carbon (2.00 g) was added to the suspension, and a reduction reaction was performed under a hydrogen atmosphere at room temperature for 5 hours. During the reaction, 50 ml in a total volume of water were gradually added. The catalyst was removed by filtration using a Kiriyama funnel (Kiriyama glass Co.) and the filtrate was concentrated under reduced pressure to a half volume. The reaction solution was further filtered through a membrane filter, and the filtrate was concentrated under reduced pressure. The residue was dissolved in water and freeze dried to obtain γ-Glu-Abu-GlyA (5.00 g, 17.2 mmol) as a white powder.
ESI-MS: $(M+H)^+$=291.10.
$^1$H-NMR (400 MHz, $D_2O$) δ (ppm): 0.86 (3H, t, J=7.40 Hz), 1.60-1.74 (1H, m), 1.82-1.88 (1H, m), 2.04-2.12 (2H, m), 2.45 (2H, t, J=7.40 Hz), 3.79 (1H, t, J=6.36 Hz), 4.31-4.45 (1H, m), 4.57 (2H, s).

[Example 8] Synthesis of γ-Glu-Val-GlyA

γ-Glu-Val-GlyA was obtained as a white powder in 77.5% yield in the same manner as that in Example 7 except that Boc-Val-OH was used in place of Boc-Abu-OH.
ESI-MS: $(M-H)^-$=303.20.
$^1$H-NMR (400 MHz, $D_2O$) δ (ppm): 0.90 (6H, t, J=6.52 Hz), 2.05-2.15 (2H, m), 2.15-2.25 (1H, m), 2.45-2.50 (2H, m), 3.80 (1H, t, J=6.52 Hz), 4.36 (1H, t, J=5.64 Hz), 4.61 (2H, s).

[Example 9] Synthesis of γ-Glu-t-Leu-GlyA

γ-Glu-t-Leu-GlyA was obtained as a white powder in 73.4% yield in the same manner as that in Example 7 except that Boc-t-Leu-OH was used in place of Boc-Abu-OH.
ESI-MS: $(M+H)^+$=319.20.
$^1$H-NMR (400 MHz, $D_2O$) 5 (ppm): 0.94 (9H, s), 2.03-2.10 (2H, m), 2.45-2.50 (2H, m), 3.78 (1H, t), 4.26 (1H, s), 4.60 (2H, s).

[Example 10] Synthesis of γ-Glu-Abu-LacA

γ-Glu-Abu-LacA was obtained as a white powder in 99.0% yield in the same manner as that in Example 7 except that benzyl (S) -lactate (H-LacA-OBzl) was used in place of benzyl glycolate (H-GlyA-OBzl).
ESI-MS: $(M+H)^+$=305.10.
$^1$H-NMR (400 MHz, $D_2O$) δ (ppm): 0.91 (3H, t, J=7.40 Hz), 1.40 (3H, d, J=7.08 Hz), 1.60-1.75 (1H, m), 1.80-1.90 (1H, m), 2.00-2.12 (2H, m), 2.40-2.45 (2H, m), 3.74-3.78 (1H, m), 4.25-4.29 (1H, m), 4.89-4.95 (1H, m).

[Example 11] Synthesis of γ-Glu-Val-LacA

γ-Glu-Val-LacA was obtained as a white powder in 78.0% yield in the same manner as that in Example 7 except that Boc-Val-OH was used in place of Boc-Abu-OH and benzyl (S)-lactate (H-LacA-OBzl) was used in place of benzyl glycolate (H-GlyA-OBzl).
ESI-MS: $(M+H)^+$=319.20.
$^1$H-NMR (400 MHz, $D_2O$) δ (ppm): 0.85-0.92 (6H, m), 1.42 (3H, d, J=7.08 Hz), 2.02-2.11 (3H, m), 2.18-2.25 (1H, m), 2.42-2.50 (2H, m), 3.78 (1H, t, J=6.36 Hz), 4.20-4.31 (1H, m), 4.91-4.97 (1H, m).

[Example 12] Synthesis of γ-Glu-t-Leu-LacA

γ-Glu-t-Leu-LacA was obtained as a white powder in 55.0% yield in the same manner as that in Example 7 except that Boc-t-Leu-OH was used in place of Boc-Abu-OH and benzyl (S) -lactate (H-LacA-OBzl) was used in place of benzyl glycolate (H-GlyA-OBzl).
ESI-MS: $(M+H)^+$=333.20.
$^1$H-NMR (400 MHz, $D_2O$) δ (ppm): 0.96 (9H, s), 1.42 (3H, d, J=7.08 Hz), 2.05-2.10 (2H, m), 2.40-2.50 (2H, m), 3.73-3.78 (1H, m), 4.19 (1H, s), 4.90-5.00 (1H, m).

[Example 13] Evaluation of calcium receptor-activating action

For evaluation of the calcium receptor-activating action, a Ca ion concentration-dependent Cl ionic current measuring method using a *Xenopus laevis* oocyte expression system was used. If each activator is added to *Xenopus*

*laevis* oocytes expressing the calcium receptor, intracellular Ca ions increase. Then, the Ca ion concentration-dependent Cl channel opens, and the intracellular current value changes as an ionic current. By measuring the change in the intracellular current value, whether the calcium receptor -activating action is present or not can be determined.

Specifically, the abdomen of *Xenopus laevis* was opened, and an egg batch was taken out and then treated with a 1% collagenase solution at 20°C for 2 hours to obtain individual oocytes. Into the oocytes, 50 nl of 1 μg/μl receptor cRNA or 50 nl of sterilized water per oocyte were injected by using a micro glass capillary, and the oocytes were cultured at 18° C for 2 to 3 days. For the culture, a solution obtained by adding 2 mM pyruvic acid, 10 U/ml penicillin, and 10 μg/ml streptomycin to the solution in Example 2 was used. After the culture, a test solution was added to the oocytes injected with cRNA or sterilized water. Electrophysiological measurement was performed by using an amplifier Geneclamp 500 (manufactured by Axon) and recording software AxoScope 9.0 (manufactured by Axon). The oocytes were membrane potential-clamped at -70 mV by the double electrode potential clamp method, and the intracellular current was measured via the Ca ion concentration-dependent Cl ion channel. The maximum value of the intracellular current was defined as the response current value.

[Example 14] Evaluation of calcium receptor-activating action of calcium

The calcium receptor-activating action of calcium was evaluated by using the method described in Example 13. That is, oocytes injected with cRNA of the calcium receptor or sterilized water were prepared, and membrane potential-clamped at -70 mV by the double electrode potential clamp method. To the potential-clamped oocytes, calcium was added (2 mM, 5 mM, 10 mM, and 20 mM), and Ca ion concentration-dependent Cl response current was measured. FIG. 1 shows the results. The results confirmed that cRNA of the calcium receptor injected into the oocytes was functionally expressed. Furthermore, since the oocytes injected with water did not respond to even a high concentration of calcium, it was confirmed that the calcium receptor was not expressed in the oocytes themselves.

[Example 15] Evaluation of calcium receptor-activating action of L-amino acids

The calcium receptor-activating action of L-amino acids was evaluated by using the method described in Example 13. That is, oocytes injected with cRNA of the calcium receptor or sterilized water were prepared, and membrane potential-clamped at -70 mV by the double electrode potential clamp method. To the potential-clamped oocytes, alanine (10 mM), arginine (10 mM), asparagine (10 mM), aspartic acid (10 mM), cysteine (10 mM), glutamine (10 mM), glutamic acid (10 mM), glycine (10 mM), histidine (10 mM), isoleucine (10 mM), leucine (10 mM), lysine (10 mM), methionine (10 mM), phenylalanine (10 mM), proline (10 mM), serine (10 mM), threonine (10 mM), tryptophan (10 mM), tyrosine (10 mM), valine (10 mM), ornithine (10 mM), taurine (10 mM), or hydroxyproline (10 mM) was added, and Ca ion concentration-dependent Cl response current was measured. FIG. 2 shows the results. The results demonstrated that cysteine, histidine, phenylalanine, tryptophan, and tyrosine each had a definite calcium receptor-activating action. As for the above-described amino acids, the activating action is reported in Proc. Natl. Acad. Sci. USA, Apr. 25, 2000, 97(9): 4814-9.

[Example 6] Evaluation of calcium receptor-activating action of D-cysteine

The calcium receptor-activating action of D-cysteine was evaluated by using the method described in Example 13. That is, oocytes injected with cRNA of the calcium receptor or sterilized water were prepared, and membrane potential-clamped at -70 mV by the double electrode potential clamp method. To the potential-clamped oocytes, D-cysteine (10 mM), L-cysteine (10 mM), D-tryptophan (10 mM), or L-tryptophan (10 mM) was added, and Ca ion concentration-dependent Cl response current was measured. FIG. 3 shows the results. The results demonstrated that D-cysteine had a definite calcium receptor-activating action.

[Example 17] Evaluation of calcium receptor-activating action of peptide

The calcium receptor-activating action of a peptide was evaluated by using the method described in Example 13. That is, oocytes injected with cRNA of the calcium receptor or sterilized water were prepared, and membrane potential-clamped at -70 mV by the double electrode potential clamp method. To the potential-clamped oocytes, γ-Glu-Cys-Gly (50 μM), γ-Glu-Cys(SNO)-Gly (50 μM), γ-Glu-Ala (50 μM), γ-Glu-Gly (500 μM), γ-Glu-Cys (50 μM), γ-Glu-Met (500 μM), γ-Glu-Thr (50 μM), γ-Glu-Val (50 μM), γ-Glu-Orn (500 μM), Asp-Gly (1 mM), Cys-Gly (1 mM), Cys-Met (1 mM), Glu-Cys (50 μM), Gly-Cys (500 μM), or Leu-Asp (1 mM) was added, and Ca ion concentration-dependent Cl response current was measured. FIG. 4 shows the results. The results demonstrated that the above-described peptide had a definite calcium receptor-activating action.

[Example 18] Evaluation of calcium receptor-activating action of peptide

The calcium receptor-activating action of a peptide was evaluated in the same manner as that of Example 17. Each of the peptides shown in Table 1 was added to potential-clamped oocytes at 1,000 μM, 300 μM, 100 μM, 30 μM, 10 μM, 3 μM, 1 μM, 0.3 μM, and 0.1 μM, and Ca ion concentration-dependent Cl response current was measured. The lowest concentration at which current was detected is shown in Table 1 as the activity. The results revealed that those 32 kinds of peptides each had a calcium receptor-activating action.

Table 1

| Number | Peptide | Activity |
|---|---|---|
| 1 | γ-Glu-Met(O) | 1,000 μM |
| 2 | γ-Glu-Val-Val | 1,000 μM |
| 3 | γ-Glu-Val-Glu | 1,000 μM |
| 4 | γ-Glu-Val-Lys | 1,000 μM |
| 5 | γ-Glu-Val-Arg | 1,000 μM |
| 6 | γ-Glu-Val-Asp | 1,000 μM |
| 7 | γ-Glu-Val-Met | 1,000 μM |
| 8 | γ-Glu-Val-Thr | 1,000 μM |
| 9 | γ-Glu-γ-Glu-Val | 1,000 μM |
| 10 | γ-Glu-Val-$NH_2$ | 1,000 μM |
| 11 | γ-Glu-Val-ol | 1,000 μM |
| 12 | γ-Glu-Ser | 300 μM |
| 13 | γ-Glu-Tau | 300 μM |
| 14 | γ-Glu-Cys(S-Me)(O) | 300 μM |
| 15 | γ-Glu-Val-His | 100 μM |
| 16 | γ-Glu-Val-Orn | 100 μM |
| 17 | γ-Glu-Leu | 100 μM |
| 18 | γ-Glu-Ile | 100 μM |
| 19 | γ-Glu-t-Leu | 100 μM |
| 20 | γ-Glu-Cys(S-allyl)-Gly | 100 μM |
| 21 | γ-Glu-Val-Asn | 30 μM |
| 22 | γ-Glu-Gly-Gly | 30 μM |
| 23 | γ-Glu-Val-Phe | 30 μM |
| 24 | γ-Glu-Val-Ser | 30 μM |
| 25 | γ-Glu-Val-Pro | 30 μM |
| 26 | γ-Glu-Ser-Gly | 30 μM |
| 27 | γ-Glu-Cys(S-Me) | 30 μM |
| 28 | γ-Glu-Val-Cys | 10 μM |
| 29 | γ-Glu-Val-Gln | 10 μM |
| 30 | γ-Glu-Abu-Gly | 3 μM |
| 31 | γ-Glu-Cys(S-Me)-Gly | 3 μM |
| 32 | γ-Glu-Val-Gly | 0.1 μM |

[Example 19] Therapeutic effect on diarrhea of peptide having calcium receptor-activating action for anticancer agent-induced diarrhea model

To each of Balb/c mice, an anticancer agent was administered to induce diarrhea, and γ-Glu-Val-Gly (hereinafter, referred to as "γEVG") was studied for its inhibitory effect on diarrhea. To each of 6-week-old Balb/c mice fed with a low protein nutrient diet (4% casein diet) for 1 week, 5-FU (1 mg/animal/day) was intraperitoneally administered for consecutive 3 days. The diarrhea symptom developed around Day 5 after the third administration of the anticancer agent, and the diarrhea symptom appeared in all cases on Day 7. The presence or absence of diarrhea was determined based on the presence or absence of the stool attachment in the tail head area. As the statistical test, a chi-square ($\chi^2$) test for the control group (presence or absence of diarrhea) was employed, and $p<0.05$ was regarded as significant.

Each administration of 0.01% γEVG was started in free water intake at the same time as the first administration of the anticancer agent, and continued until completion of the experiment.

All (10/10) of the mice exhibited the diarrhea symptom in the control group, while 2 out of 5 mice did not exhibit the diarrhea symptom in the 0.01% γEVG administration group. Those results indicate that γEVG has a significant therapeutic effect on diarrhea in the anticancer agent-induced diarrhea model.

Table 2 γEVG administration effect on diarrhea model mice

| Medicament | Ratio of individuals that developed diarrhea |
|---|---|
| Control group | 10/10 |
| 0.01% γEVG | 3/5* |
| * p<0.05 (chi-square test) | |

[Example 20] Therapeutic effect on diarrhea of peptide having calcium receptor-activating action in mice 5-HTP-induced defecation model

Male ICR mice (5-week-old) were used. To each of the mice, γEVG, which had been dissolved in a 0.5% carboxymethylcellulose aqueous solution, was orally administrated, and after 1 hour, 5-HTP (5-hydroxy tryptophan, 10 mg/kg and 5 ml/kg) was subcutaneously administered. After 30 minutes, the stool form score (0: normal stool or no stool and 1: diarrhea or loose stool) was measured. As a control, a vehicle (0.5% carboxymethylcellulose aqueous solution) free of any medicament was administered to each of the mice.

γEVG was prepared so that the concentrations would be 1% and 0.1% (w/v, hereafter, interpreted with the same meaning).

As the statistical test, a chi-square ($\chi^2$) test for the vehicle administration group (presence or absence of diarrhea and loose stool) was employed, and $p<0.05$ was regarded as significant.

FIG. 5 shows the results. The stool form score of the vehicle administration group significantly increased compared with that of a healthy group. The administration of 0.1% or 1% γEVG improved the stool form score dose-dependently. In addition, 1% γEVG improved diarrhea significantly.

[Example 21] Influence of CaSR activator on fluid absorption in rat large intestine loop method

<Method>

The cecum and large intestine were extirpated from the abdomen of each of male SD (IGS) rats under pentobarbital anesthesia, and the site 5 cm away from the area just under the cecum was ligated to form a large intestine loop. Immediately after the loop had been formed, PGE2 (4 μg/ml/kg, SIGMA) was intraperitoneally administered, and after 30 minutes, a medicament, which had been dissolved in 2 ml of Tyrode's solution (NaCl: 136.9 mM, KCl: 2.7 mM, $CaCl_2 \cdot 2H_2O$: 1.8 mM, $MgCl_2 \cdot H_2O$: 1.04 mM, $NaH_2PO_4 \cdot 2H_2O$: 0.04 mM, $NaH_2PO_4 \cdot 2H_2O$: 0.04 mM, glucose: 5.55 mM, $NaHCO_3$: 11. 9 mM), was injected into the prepared loop (the medicament solution was adjusted so that the pH would be 6.5 to 7.5). As a control, Tyrode' s solution free of any medicament (vehicle) was administered. After 1 hour, the loop weight, the loop weight after solution removal, and the loop area were measured to calculate a solution weight per unit area remaining in the loop.

The remaining solution amount per unit area was calculated with the following equation:

Remaining solution amount per unit area ($g/cm^2$) = (Loop weight - Loop weight after solution removal)/Loop area.

The fluid absorption was evaluated by calculating an inhibitory rate from the following equation:

Inhibitory rate (%) = 100 - (Remaining solution amount per unit area under drug administration - Remaining solution amount per unit area in base (average))/(Remaining solution amount per unit area under vehicle administration (average) - Remaining solution amount per unit area in base (average)) × 100.

(Base = case where no stimulation is given (no treatment with PGE2))

FIGS. 6 to 8 show the results. γEVG promoted the fluid absorption in a dose-dependent manner. Further, GSH, γ-Glu-t-Leu-Gly, and cinacalcet also promoted the fluid absorption. Thus, it was found that the fluid absorption promoted by those compound groups provided an inhibitory effect on diarrhea.

[Example 22] Diarrhea inhibitory effect of calcium receptor activator for anticancer agent-induced diarrhea model

To each of Balb/c mice, an anticancer agent was administered to induce diarrhea, and a calcium receptor activator (hereinafter, referred to as "CaSR agonist") was studied for its inhibitory effect on diarrhea. To each of 6-week-old Balb/c mice fed with a low protein nutrient diet (4% casein diet) for 1 week, 5-FU (1 mg/animal/day) was intraperitoneally administered for consecutive 3 days. The diarrhea symptom developed around Day 5 after the third administration of the anticancer agent, and the diarrhea symptom appeared in all cases on Day 7. The presence or absence of diarrhea was determined based on the presence or absence of the stool attachment in the tail head area. As the statistical test, a chi-square ($\chi^2$) test for the control group (presence or absence of diarrhea) was employed, and $p<0.05$ was regarded as significant.

The administration of the CaSR agonist was started in free water intake at the same time as the administration of the low protein nutrient diet, and continued until completion of the experiment.

All (9/9) of the mice exhibited the diarrhea symptom in the control group, while 2 out of 5 mice did not exhibit the diarrhea symptom in both the 0.05% cinacalcet administration group and the 0.5% γ-Glu-Cys-Gly administration group. Those results indicate that the CaSR agonist has a significant inhibitory effect on diarrhea in the anticancer agent-induced diarrhea model.

Table 3

| Medicament | Ratio of individuals that developed diarrhea |
|---|---|
| Control group | 9/9 |
| 0.05% cinacalcet | 3/5* |
| 0.5% γ-Glu-Cys-Gly | 3/5* |
| * $p<0.05$ $\chi^2$ test for control group (presence or absence of diarrhea) | |

[Example 23] Measurement of activity of CaSR agonist peptide

Various peptides synthesized in Example 2 and Examples 6 to 10 were measured for their activities as follows. The cDNA of the human CaSR obtained by the method described in Example 1 of Patent Document WO 2007/055393 A1 was incorporated into an expression vector for animal cells having a CMV promoter, and the gene was introduced into HEK 293 cells which grew up to about 70% of the maximum cell density by using FuGINE 6 (F. Hoffmann-La Roche,

Ltd.). After the culture for 4 to 48 hours, the cells were seeded into a 96-well plate in an amount of 0.6 to 1.0×$10^5$ cells/well, and cultured for 1 day. After that, the cells were stained with a Calcium 3 assay kit. The presence or absence of the activity of the CaSR agonist peptide was confirmed by a calcium imaging method using FLEXSTATION (MDS Inc. , instruction manual). In addition, the concentration that provides 50% of the maximum activity was determined as EC50 from a dose characteristic curve.

Table 4

| Compounds | EC50, μM |
|---|---|
| γ-Glu-Val-Gly | 0.023-0.055 |
| γ-Glu-Cle-Gly | 0.5-1.5 |
| γ-Glu-Aib-Gly | 0.349-1.167 |
| γ-Glu-t-Leu-Gly | 0.043-0.077 |
| γ-Glu-Pen-Gly | 0.159-0.233 |
| γ-Glu-Val-GlyA | 0.14 |
| γ-Glu-t-Leu-GlyA | 0.05 |
| γ-Glu-Abu-GlyA | 0.065-0.079 |
| γ-Glu-Val-LacA | 0.057 |
| γ-Glu-t-Leu-LacA | 0.038 |
| γ-Glu-Abu-LacA | 0.102 |
| *The experimental value including only one numerical value indicates a value with n=1, and the other experimental values indicate a range of values with n=2 to 3. | |

Industrial Applicability

The present invention provides a prophylactic or therapeutic agent for diarrhea, which is highly safe to the living body.

## Claims

1. An agent comprising a compound having calcium receptor-activating action for use in a method for prophylactic or therapeutic treatment of diarrhea,
wherein the compound is selected from a peptide or a peptide derivative,
wherein the peptide is one kind or two or more kinds selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys (S-Me), and
wherein the peptide derivative has a structure of the following formula (3):

$$\gamma\text{-Glu-X-OCH(Z)}CO_2H \qquad (3)$$

wherein X represents an amino acid or an amino acid derivative and Z represents H or $CH_3$.

2. The agent for use according to claim 1, wherein X represents Cys(SNO), Cys(S-allyl), Gly, Cys(S-Me), Cys, Abu, t-Leu, Cle, Aib, Pen or Ser, and Y represents Gly, Val, Glu, Lys, Phe, Ser, Pro, Arg, Asp, Met, Thr, His, Orn, Asn, Cys or Gln.

3. The agent for use according to claim 1 or 2, wherein the peptide is γ-Glu-Val-Gly or γ-Glu-t-Leu-Gly.

4. A compound, which has a structure of the following formula (3):

$$\gamma\text{-Glu-X-OCH(Z)}C0_2H \qquad (3)$$

wherein X represents an amino acid or an amino acid derivative and Z represents $CH_3$; or
wherein X represents t-Leu or Abu and Z represents H.

5. A compound according to claim 4, wherein X represents t-Leu or Abu and Z represents $CH_3$.

6. A compound according to claim 4 or 5, which is selected from the group consisting of of γ-Glu-Val-GlyA, γ-Glu-t-Leu-GlyA, γ-Glu-Abu-GlyA, γ-Glu-Val-LacA, γ-Glu-t-Leu-LacA, and γ-Glu-Abu-LacA.

7. A compound, which is represented by γ-Glu-t-Leu-Gly.

## Patentansprüche

1. Mittel, welches eine Verbindung mit Kalziumrezeptoraktivierungswirkung umfasst, für die Verwendung in einem Verfahren zur vorbeugenden oder therapeutischen Behandlung von Durchfall, wobei die Verbindung unter einem Peptid oder einem Peptidderivat ausgewählt ist, wobei das Peptid eine Art oder zwei oder mehr Arten darstellt, ausgewählt aus der Gruppe, die aus γ-Glu-X-Gly (X stellt eine Aminosäure oder ein Aminosäurederivat dar), γ-Glu-Val-Y (Y stellt eine Aminosäure oder ein Aminosäurederivat dar), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-$NH_2$, γ-Glu-Val-ol, γ-Glu-Ser, γ-Glu-Tau, γ-Glu-Cys(S-Me) (O), γ-Glu-Leu, γ-Glu-Ile, γ-Glu-t-Leu, und γ-Glu-Cys(S-Me) besteht, und
wobei das Peptidderivat eine Struktur der folgenden Formel (3) aufweist:

γ-Glu-X-OCH(Z)$CO_2$H (3)

wobei X eine Aminosäure oder ein Aminosäurederivat darstellt und Z H oder $CH_3$ darstellt.

2. Mittel für die Verwendung nach Anspruch 1, wobei X Cys(SNO), Cys(S-allyl), Gly, Cys(S-Me), Cys, Abu, t-Leu, Cle, Aib, Pen, oder Ser darstellt, und Y Gly, Val, Glu, Lys, Phe, Ser, Pro, Arg, Asp, Met, Thr, His, Orn, Asn, Cys oder Gln darstellt.

3. Mittel zur Verwendung nach Anspruch 1 oder 2, wobei das Peptid γ-Glu-Val-Gly oder γ-Glu-t-Leu-Gly ist.

4. Verbindung, die eine Struktur der folgenden Formel (3) aufweist:

γ-Glu-X-OCH(Z)$CO_2$H (3)

wobei X eine Aminosäure oder ein Aminosäurederivat darstellt und Z $CH_3$ darstellt; oder
wobei X t-Leu oder Abu darstellt und Z H darstellt.

5. Verbindung nach Anspruch 4, wobei X t-Leu oder Abu darstellt und Z $CH_3$ darstellt.

6. Verbindung nach Anspruch 4 oder 5, die aus der Gruppe ausgewählt ist, die aus γ-Glu-Val-GlyA, γ-Glu-t-Leu-GlyA, γ-Glu-Abu-GlyA, γ-Glu-Val-LacA, γ-Glu-t-Leu-LacA und γ-Glu-Abu-LacA besteht.

7. Verbindung, die durch γ-Glu-t-Leu-Gly dargestellt ist.

## Revendications

1. Agent comprenant un composé ayant une action d’activation des récepteurs du calcium pour une utilisation dans un procédé de traitement prophylactique ou thérapeutique de la diarrhée,
dans lequel le composé est choisi parmi un peptide ou un dérivé peptidique,
dans lequel le peptide est un type ou deux ou plusieurs types choisis dans le groupe constitué de γ-Glu-X-Gly (X représente un acide aminé ou un dérivé d’acide aminé), de γ-Glu-Val-Y (Y représente un acide aminé ou un dérivé d’acide aminé), de γ-Glu-Ala, de γ-Glu-Gly, de γ-Glu-Cys, de γ-Glu-Met, de γ-Glu-Thr, de γ-Glu-Val, de γ-Glu-Orn, d’Asp-Gly, de Cys-Gly, de Cys-Met, de Glu-Cys, de Gly-Cys, de Leu-Asp, de γ-Glu-Met(O), de γ-Glu-γ-Glu-Val, de γ-Glu-Val-$NH_2$, de γ-Glu-Val-ol, de γ-Glu-Ser, de γ-Glu-Tau, de γ-Glu-Cys(S-Me)(O), de γ-Glu-Leu, de γ-Glu-Ile, de γ-Glu-t-Leu et de γ-Glu-Cys(S-Me), et
dans lequel le dérivé peptidique a une structure de la formule suivante (3) :

γ-Glu-X-OCH(Z)$CO_2$H (3)

dans laquelle X représente un acide aminé ou un dérivé d’acide aminé et Z représente un atome d’H ou un groupe $CH_3$.

2. Agent pour une utilisation selon la revendication 1, dans lequel X représente Cys(SNO), Cys(S-allyle), Gly, Cys(S-Me), Cys, Abu, t-Leu, Cle, Aib, Pen ou Ser, et Y représente Gly, Val, Glu, Lys, Phe, Ser, Pro, Arg, Asp, Met, Thr, His, Orn, Asn, Cys ou Gln.

3. Agent pour une utilisation selon la revendication 1 ou 2, dans lequel le peptide est γ-Glu-Val-Gly ou γ-Glu-t-Leu-Gly.

4. Composé, qui a une structure de la formule suivante (3) :

$$\gamma\text{-Glu-X-OCH(Z)}CO_2H \qquad (3)$$

dans laquelle X représente un acide aminé ou un dérivé d'acide aminé et Z représente un groupe $CH_3$ ; ou dans laquelle X représente t-Leu ou Abu et Z représente un atome d'H.

5. Composé selon la revendication 4, dans lequel X représente t-Leu ou Abu et Z représente un groupe $CH_3$.

6. Composé selon la revendication 4 ou 5, qui est choisi dans le groupe constitué de γ-Glu-Val-GlyA, de γ-Glu-t-Leu-GlyA, de γ-Glu-Abu-GlyA, de γ-Glu-Val-LacA, de γ-Glu-t-Leu-LacA et de γ-Glu-Abu-LacA.

7. Composé, qui est représenté par γ-Glu-t-Leu-Gly.

Fig.1

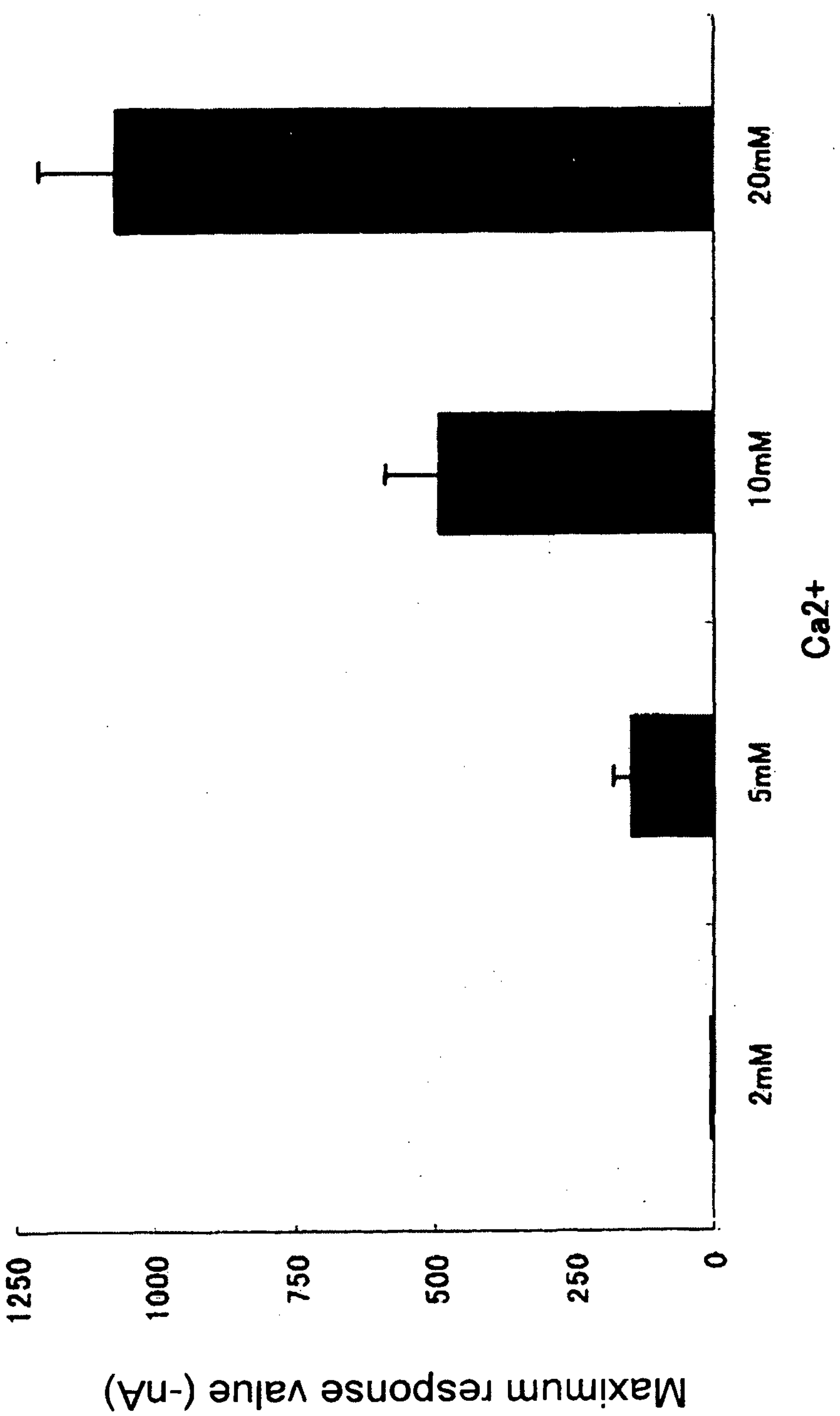
Maximum response value (-nA)
1250
1000
750
500
250
0
2mM
5mM
10mM
20mM
Ca2+

Fig.2

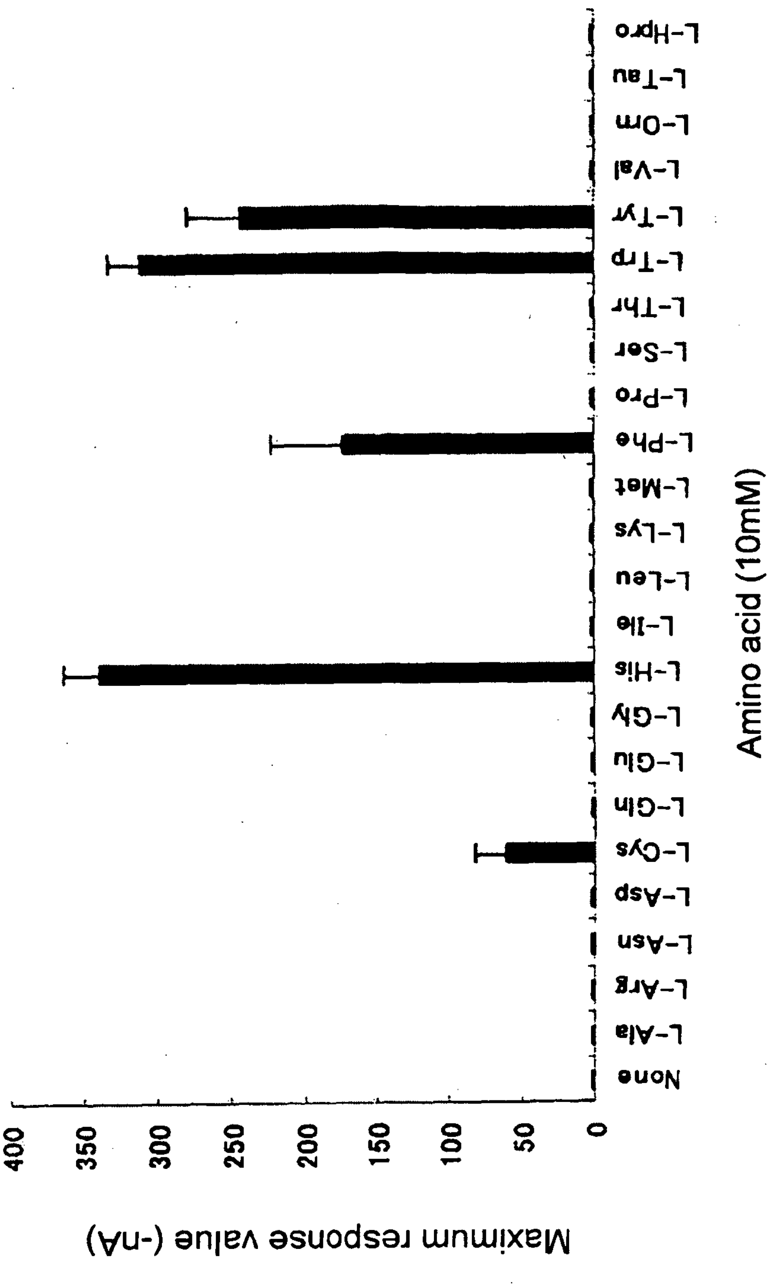
Maximum response value (-nA)
0
50
100
150
200
250
300
350
400
None
L-Ala
L-Arg
L-Asn
L-Asp
L-Cys
L-Gln
L-Glu
L-Gly
L-His
L-Ile
L-Leu
L-Lys
L-Met
L-Phe
L-Pro
L-Ser
L-Thr
L-Trp
L-Tyr
L-Val
L-Om
L-Tau
L-Hpro
Amino acid (10mM)

Fig.3

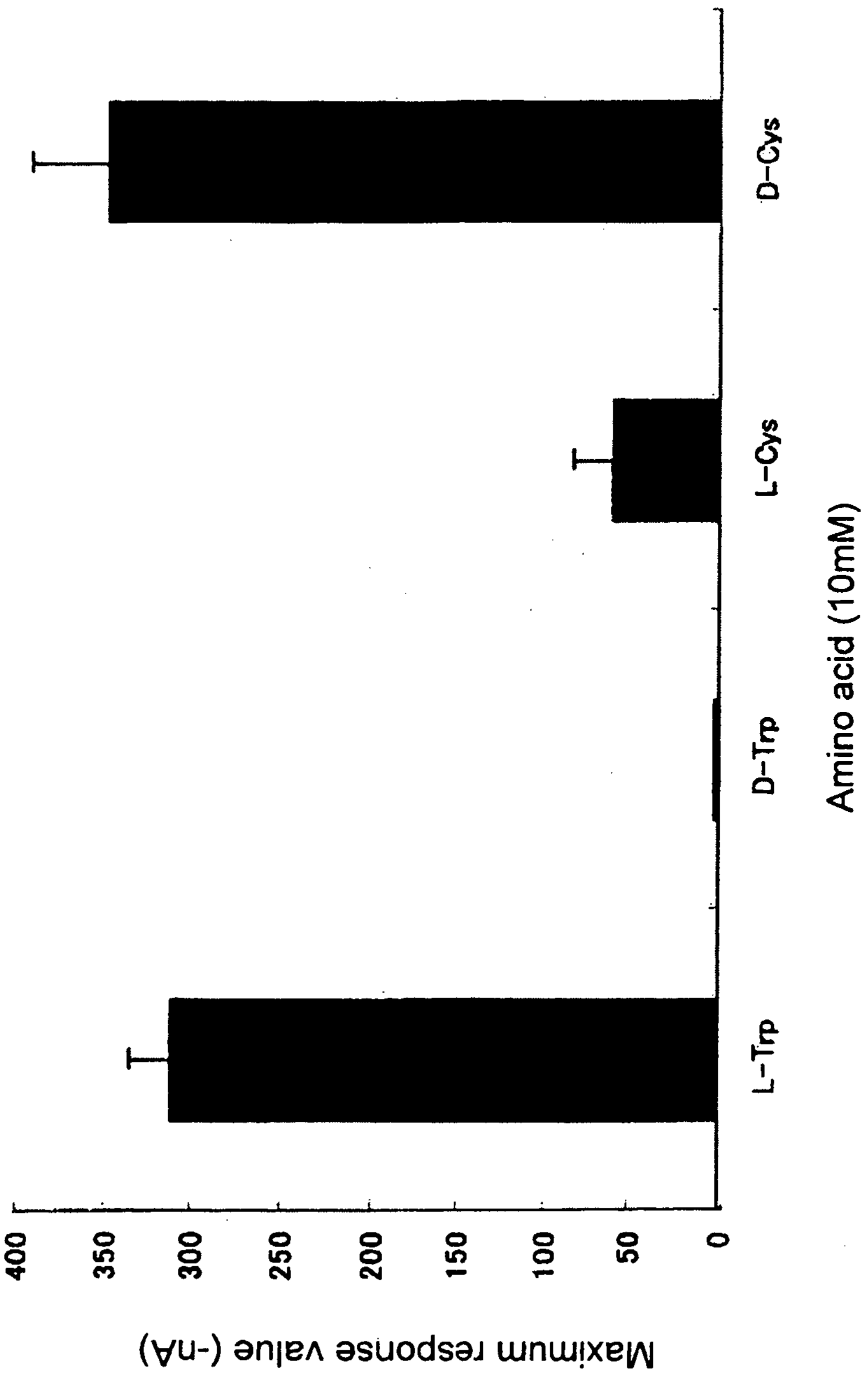
Maximum response value (-nA)
0
50
100
150
200
250
300
350
400
L-Trp
D-Trp
L-Cys
D-Cys
Amino acid (10mM)

Fig.4

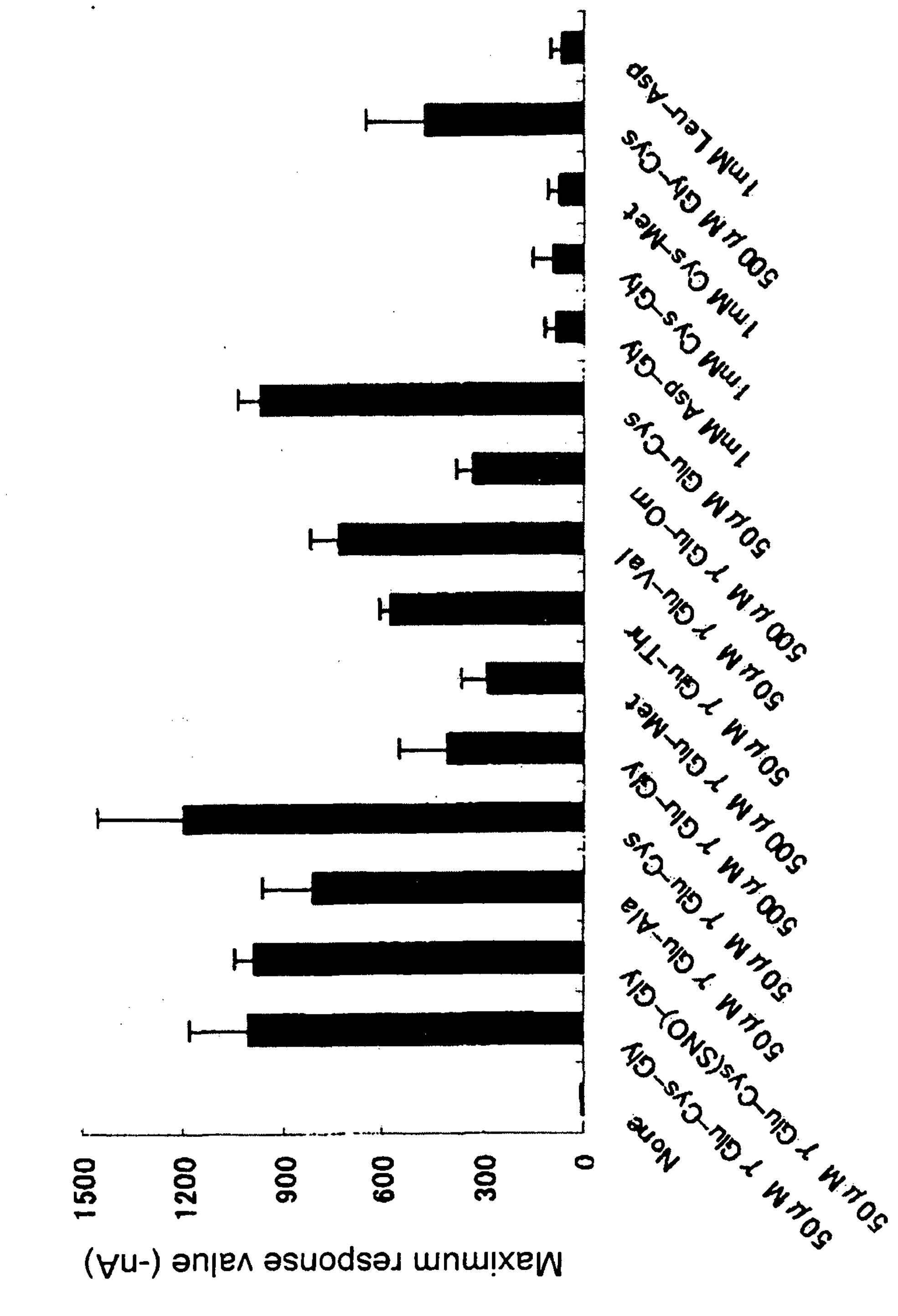
Maximum response value (-nA)
1500
1200
900
600
300
0
None
50 μM γ Glu-Cys-Gly
500 μM γ Glu-Cys(SNO)-Gly
50 μM γ Glu-Ala
50 μM γ Glu-Cys
500 μM γ Glu-Gly
500 μM γ Glu-Met
50 μM γ Glu-Thr
500 μM γ Glu-Val
50 μM γ Glu-Orn
500 μM Glu-Cys
1mM Asp-Gly
1mM Cys-Gly
1mM Cys-Met
500 μM Gly-Cys
1mM Leu-Asp

Fig.5

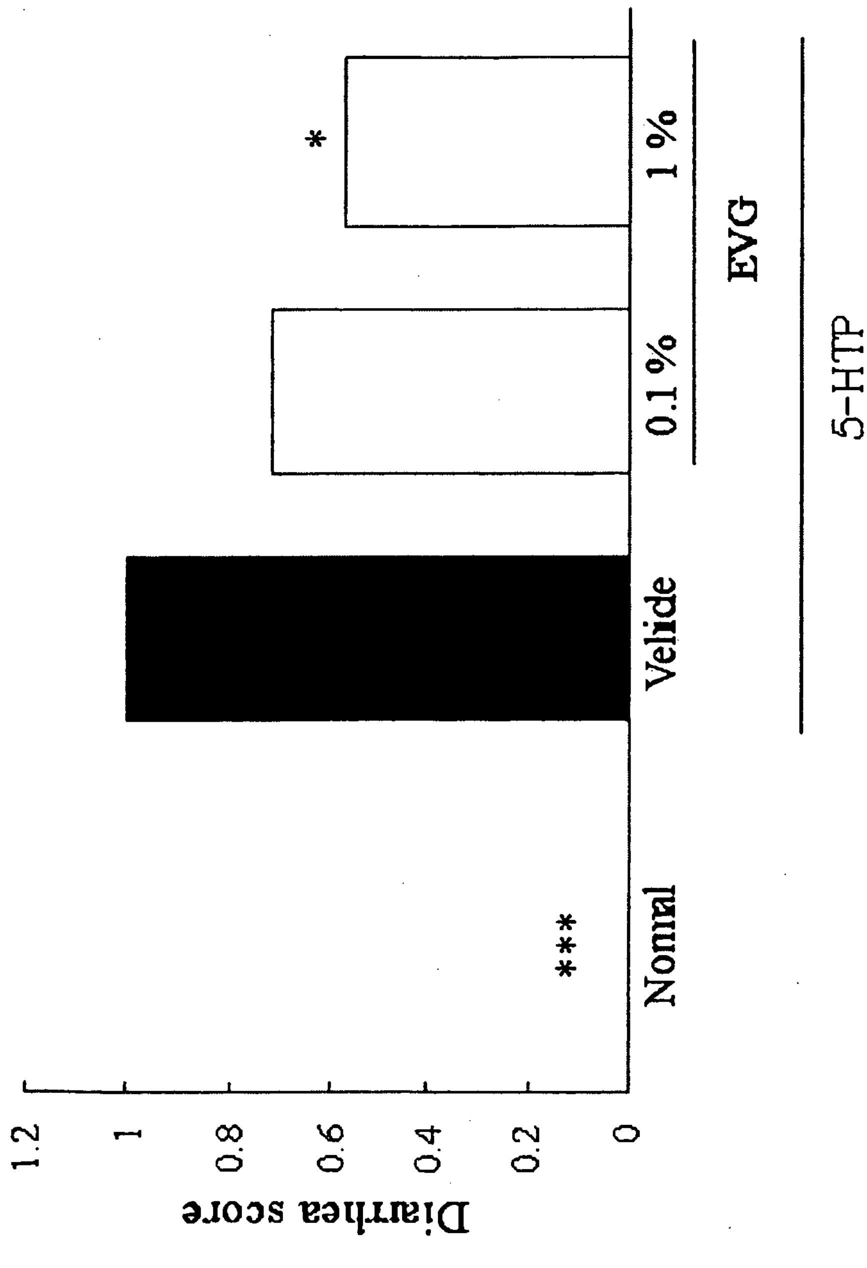

Fig.6

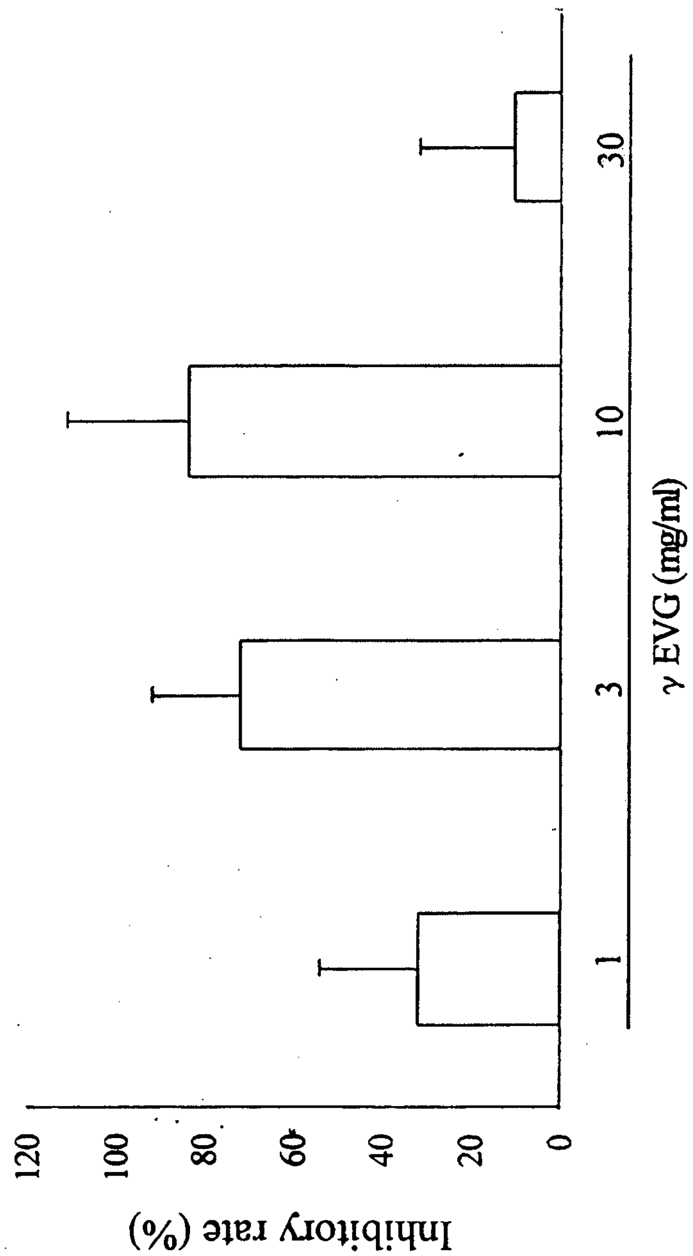

Fig.7

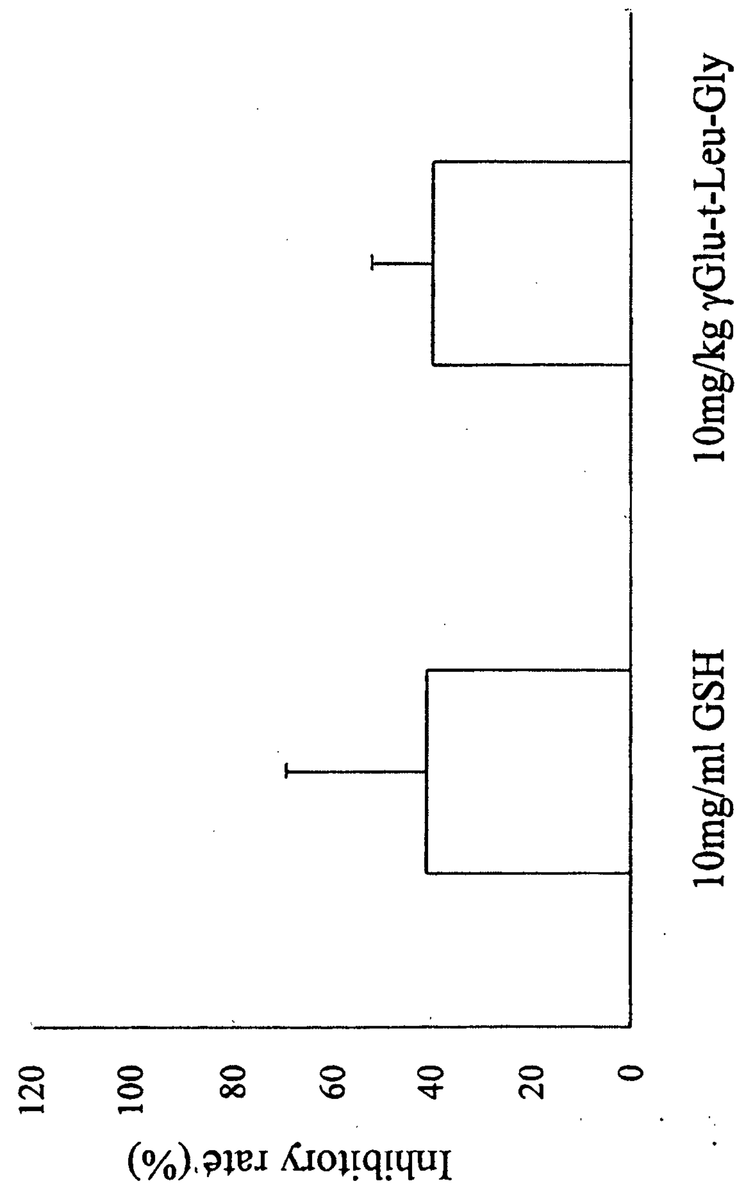

Inhibitory rate (%)
120
100
80
60
40
20
0
10mg/ml GSH
10mg/kg γGlu-t-Leu-Gly

Fig.8

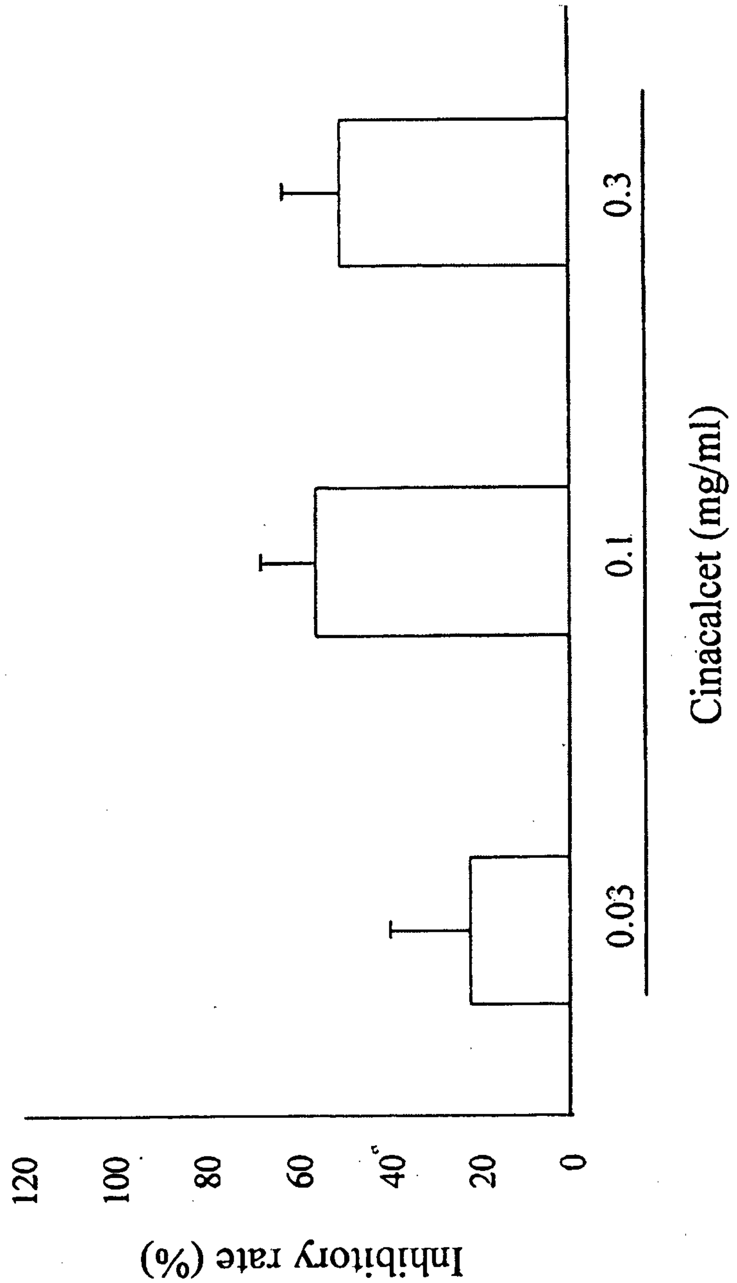

Inhibitory rate (%)
120
100
80
60
40
20
0
0.03
0.1
0.3
Cinacalcet (mg/ml)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004011653 A
- WO 2007055393 A1
- EP 64832 A
- JP 2007123765 A


### Non-patent literature cited in the description


- *Nature,* 1993, vol. 366 (6455), 575-580
- *J. Endocrinol.,* 2000, vol. 165 (2), 173-177
- *Eur. J. Pharmacol.,* 2002, vol. 447 (2-3), 271-278
- *Cell Calcium,* 2004, vol. 35 (3), 209-216
- *J. Biol. Chem.,* 2006, vol. 281 (13), 8864-8870
- *Proc. Natl. Acad. Sci. USA,* 2006, vol. 103 (25), 9390-9397
- *Proc. Natl. Acad. Sci. USA,* 25 April 2000, vol. 97 (9), 4814-9

SEQUENCE LISTING

<110> Ajinomoto Co., Inc.

<120> PROPHYLACTIC OR THERAPEUTIC AGENT FOR DIARRHEA

<130> EPA-64832

<150> JP2007-123765
<151> 2007-05-08

<160> 2

<170> PatentIn version 3.1

<210> 1
<211> 49
<212> DNA

<213> Artificial

<220>
<223> hCASR_N primer

<400> 1
actaatacga ctcactatag ggaccatggc attttatagc tgctgctgg 49

<210> 2
<211> 29
<212> DNA
<213> Artificial

<220>
<223> HCASR_C primer

<400> 2
ttatgaattc actacgtttt ctgtaacag 29